(19) Europäisches Patentamt / European Patent Office / Office européen des brevets

(11) **EP 2 995 300 B1**

(12) **EUROPEAN PATENT SPECIFICATION**

(45) Date of publication and mention of the grant of the patent:
**22.04.2020 Bulletin 2020/17**

(21) Application number: **13875242.3**

(22) Date of filing: **19.12.2013**

(51) Int Cl.:
*A61K 47/54* (2017.01)     *A61K 47/69* (2017.01)
*A61P 35/00* (2006.01)     *A61K 38/00* (2006.01)
*C12N 15/115* (2010.01)

(86) International application number:
**PCT/KR2013/011851**

(87) International publication number:
**WO 2014/126332 (21.08.2014 Gazette 2014/34)**

(54) **PROTEIN TRANSDUCTION DOMAIN BASED ON GOLD NANOPARTICLE-APTAMER CONJUGATE AND METHOD FOR PRODUCING SAME**

PROTEINTRANSDUKTIONSDOMÄNE AUF DER BASIS EINES GOLDNANOPARTIKELAPTAMERKONJUGATS UND VERFAHREN ZUR HERSTELLUNG DAVON

DOMAINE DE TRANSDUCTION PROTÉIQUE FONDÉ SUR UN CONJUGUÉ D'APTAMÈRES-NANOPARTICULES D'OR ET PROCÉDÉ DE PRODUCTION CORRESPONDANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.02.2013 KR 20130015552**
**18.12.2013 KR 20130158104**

(43) Date of publication of application:
**16.03.2016 Bulletin 2016/11**

(73) Proprietor: **Chung-Ang University Industry-Academic Cooperation Foundation**
**Seoul 156-756 (KR)**

(72) Inventors:
• LEE, Kangseok
  Gwangju-si
  Gyeonggi-do 464-874 (KR)
• BAE, Jeehyeon
  Gwangju-si
  Gyeonggi-do 464-874 (KR)
• SEONG, Maeng Je
  Seoul 137-907 (KR)
• HAN, Min Su
  Seoul 156-713 (KR)
• YEOM, Ji Hyun
  Yangju-si
  Gyeonggi-do 482-861 (KR)
• RYOU, Sang Mi
  Seoul 110-054 (KR)
• HA, Nam Chul
  Busan 609-735 (KR)

(74) Representative: **Dehns**
**St. Bride's House**
**10 Salisbury Square**
**London EC4Y 8JD (GB)**

(56) References cited:
WO-A1-2012/167173     KR-A- 20110 050 338
US-A1- 2007 134 665     US-A1- 2010 173 347
US-A1- 2013 022 538

• ÖZNUR KÖKPINAR ET AL: "Aptamer-based downstream processing of his-tagged proteins utilizing magnetic beads", BIOTECHNOLOGY AND BIOENGINEERING, vol. 108, no. 10, 12 October 2011 (2011-10-12), pages 2371-2379, XP055137091, ISSN: 0006-3592, DOI: 10.1002/bit.23191
• DANIELS D A ET AL: "Generation of RNA Aptamers to the G-Protein-Coupled Receptor for Neurotensin, NTS-1", ANALYTICAL BIOCHEMISTRY, ACADEMIC PRESS INC, NEW YORK, vol. 305, no. 2, 15 June 2002 (2002-06-15) , pages 214-226, XP027227022, ISSN: 0003-2697 [retrieved on 2002-06-15]

- GHOSH P ET AL: "Gold nanoparticles in delivery applications", ADVANCED DRUG DELIVERY REVIEWS, ELSEVIER, AMSTERDAM, NL, vol. 60, no. 11, 17 August 2008 (2008-08-17), pages 1307-1315, XP022849514, ISSN: 0169-409X, DOI: 10.1016/J.ADDR.2008.03.016 [retrieved on 2008-04-10]
- JOSHI HRUSHIKESH M ET AL: "Gold nanoparticles as carriers for efficient transmucosal insulin delivery", LANGMUIR, AMERICAN CHEMICAL SOCIETY, US, vol. 22, no. 1, 3 January 2006 (2006-01-03), pages 300-305, XP002591652, ISSN: 0743-7463, DOI: 10.1021/LA051982U [retrieved on 2005-12-03]
- LEE, J. H. ET AL.: 'Molecular diagnostic and drug delivery agents based on aptamer-nanomaterial conjugates' ADVANCED DRUG DELIVERY REVIEWS vol. 62, 2010, pages 592 - 605, XP027076627
- YANG, L. ET AL.: 'Aptamer-conjugated nanomaterials and their applications' ADVANCED DRUG DELIVERY REVIEWS vol. 63, 2011, pages 1361 - 1370, XP028114526
- YEOM JI-HYUN ET AL: "Gold nanoparticle-DNA aptamer conjugate-assisted delivery of antimicrobial peptide effectively eliminates intracellularSalmonella entericaserovar Typhimurium", BIOMATERIALS, ELSEVIER SCIENCE PUBLISHERS BV., BARKING, GB, vol. 104, 8 July 2016 (2016-07-08), pages 43-51, XP029681210, ISSN: 0142-9612, DOI: 10.1016/J.BIOMATERIALS.2016.07.009

Remarks:

The file contains technical information submitted after the application was filed and not included in this specification

# Description

[Technical Field]

The present invention relates to a protein delivery carrier with improved intracellular protein delivery capability by conjugating gold nanoparticles with a variety of tag aptamers or protein-specific aptamers, and a method of preparing the same.

[Background Art]

Delivery of probes or therapeutic agents to specific cells or intracellular organelles is very important for molecular-level diagnostic and therapeutic applications. Therefore, various studies for delivering target materials such as genes, proteins or drug agents past through cellular membranes to inner nuclear membrane by overcoming obstacles present in the cell has been extensively studied (Reference: Yu Jin Kim, Sang-Mi Ryou, Sudeok Kim, Ji-Hyun Yeom, Min Su Han, Kangseok Lee, Maeng-Je Seong, Enhanced protein-mediated binding between oligonucleotide gold nanoparticle composites and cell surfaces: co-transport of proteins and composites, J. Mater. Chem., 2012, 22, 25036).

Meanwhile, nanoparticles have been a promising tool for biomedical applications such as a delivery agent for drug and DNAs, intracellular imaging, or light therapy. Gold nanomaterials in particular, have been attracting much attention as an excellent candidate for such applications, due to the ease of its synthesis and action, chemical stability, biocompatibility, and adjustability in its optical and electrical properties.

However, substances prepared for diagnostic or therapeutic applications generally need to be delivered to the cell interior in a diagnostically or therapeutically significant amount, but cells are usually impermeable to macromolecules like proteins, thus such protein-based diagnostic or therapeutic techniques might face a limitation.

To overcome this problem, various techniques such as electroporation, membrane fusion using liposomes, direct single-cell microinjection have been developed, in order to deliver macromolecules like proteins to the cell interior by activating them outside or on the surface of the targeted cell. However, these techniques are shown to have an ineffective intracellular delivery capability, delivering only to some of the targeted cells and having an adverse effect on a number of untargeted cells as well.

US 2007/134665 discloses a method for the detection and localization of proteins in situ. The method comprises contacting a peptide tag labelled protein with a nucleic acid aptamer that recognizes the peptide tag. The nucleic acid aptamer is functionalized such that is detectable by a variety of methods. The method allows for the detection of one or more proteins and/or protein complexes in sub-cellular and sub-nuclear structures with high specificity and also allows for the purification of such identified proteins.

Oznur Kökpinar et al., (2011) Biotechnology and Bioengineering 108(10): 2371-2379 discloses aptamer-based downstream processing of his-tagged proteins utilizing magnetic beads.

Daniels et al., (2002) Analytical Biochemistry 305(2): 214-226 discloses generation of RNA aptamers to the G-protein-coupled-receptor for Neurotensin, NTS-1.

Ghosh et al., (2008) Advanced Drug Delivery Reviews 60(11): 1307-1315 gold nanoparticles as non-toxic carriers for drug and gene delivery applications. Disclosed is functionalized gold nanoparticles for protein delivery, gold nanoparticle targeting *in vivo,* and non-covalent and covalent conjugation of nucleic acids to gold nanoparticles.

Joshi et al., (2006) Langmuir, American Chemical Society 22(1): 300-305 discloses the loading of insulin onto bare gold nanoparticles and aspartic acid-capped gold particles and delivery to rats by oral and intranasal routes.

[Technical Problem]

To address the above-mentioned problem, the objective of the present invention is to provide a protein delivery carrier including a gold nanoparticle and an aptamer that binds to a surface of the gold nanoparticle.

Also, another objective of the present invention is to provide a method of preparing a protein delivery carrier, wherein the method includes a process of conjugating a gold nanoparticle with an aptamer.

[Technical Solution]

The present invention is as defined in the appended claims. The present invention provides a protein delivery carrier comprising a gold nanoparticle and an aptamer that conjugated to a surface of the gold nanoparticle, wherein the aptamer is selected from an aptamer that has SEQ ID NO: 1 and can specifically bind to a histidine-tag and/or an aptamer that has SEQ ID NO: 2 and can specifically bind to a glutathione S-transferase (GST)-tag.

According to an embodiment of the present invention, the gold nanoparticle may have a size of 10 to 20 nm.

According to a disclosure, the aptamer may specifically bind to a protein to be delivered.

According to still another disclosure, the protein may be a protein labeled with a tag.

According to yet another disclosure, the aptamer may bind specifically to the tag.

According to yet another disclosure, the tag may be histidine and/or glutathione S-transferase (GST).

Also, the present invention provides a method of preparing a protein delivery carrier, comprising conjugating a gold nanoparticle with one or more aptamers, wherein the one or more aptamers are selected from an aptamer that has SEQ ID NO: 1 and can specifically bind to a histidine-tag and/or an aptamer that has SEQ ID NO: 2 and can specifically bind to a glutathione S-transferase (GST)-tag.

Further, disclosed is a complex formed by conjugating a protein delivery carrier with a protein.

Furthermore, disclosed is a a protein delivery carrier for use in a method of delivering a protein, including administering a complex to an individual.

[Advantageous Effects]

A protein delivery carrier according to the present invention can effectively deliver a protein into cells by specifically binding a variety of tagged aptamers or protein-specific aptamers conjugated with a gold nanoparticle to a protein to be delivered. Also, the protein delivery carrier is harmless to the human body by using gold nanoparticles with a very low cytotoxicity and expected to be useful for diagnosis or therapy of a disease by reflecting light in various wavelengths to easily detect a location in the cells.

[Description of Drawings]

FIG. 1 is a schematic diagram of the formation of aptamer-gold nanoparticle-protein complex by conjugating His-aptamer and His-tagged protein to a gold nanoparticle.

FIG. 2 shows the results of confocal microscopy analysis of intracellular delivery of E.coli AcrA protein with Rabbit488 (Green)-labeled secondary antibodies.

FIG. 3 shows the results of confocal microscopy analysis of intracellular delivery of E.coli AcrA protein with Rabbit584 (Red)-labeled secondary antibodies.

FIG. 4 shows the results of confocal microscopy analysis of intracellular delivery of AcrA protein when HeLa cells are treated with AcrA or His-AcrA proteins which are conjugated or not conjugated with AuNP-His-Apt.

FIG. 5 shows the results of Western blot analysis performed to detect E.coli AcrA protein.

FIG. 6 shows the results of Western blot analysis of intracellular delivery of AcrA proteins when HeLa cells are treated with AcrA or His-AcrA proteins which are conjugated or not conjugated with AuNP-His-Apt.

FIG. 7 shows the results of confocal microscopy analysis of intracellular delivery of BCL-xL protein with Rabbit488 (Green)-labeled secondary antibodies.

FIG. 8 shows the results of confocal microscopy analysis of intracellular delivery of BCL-xL protein when HeLa cells are treated with BCL-xL or His-BCL-xL which are conjugated or not conjugated with AuNP-His-Apt.

FIG. 9 shows the results of Western blot analysis performed to detect BCL-xL protein.

FIG. 10 shows the results of Western blot analysis of intracellular delivery of BCL-xL protein when HeLa cells are treated with BCL-xL or His-BCL-xL which are conjugated or not conjugated with AuNP-His-Apt.

FIG. 11 shows the results of flow cytometry analysis of intracellular delivery of Alexa488-labeled BCL-xL protein.

FIG. 12 shows the results of flow cytometry analysis of intracellular delivery of Alexa488 (Green)-labeled BCL-xL protein by a Cy5 (Red)-labeled His-aptamer gold nanoparticle delivery carrier.

FIG. 13 shows the results of flow cytometry analysis of intracellular delivery of BCL-xL protein when HeLa cells are treated with BCL-xL or His-BCL-xL proteins which are conjugated or not conjugated with AuNP-His-Apt.

FIG. 14 shows the results of confocal microscopy analysis of intracellular delivery of FOXL2 protein with Rabbit-Rodamine (Red)-labeled secondary antibodies.

FIG. 15 shows the results of Western blot analysis performed to detect FOXL2 protein.

FIG. 16 shows the results of confocal microscopy analysis of HeLa cells treated and incubated with AuNP-GST-Apt-GST-FOXL2 to confirm GST-FOXL2 protein internalization mediated by AuNP-GST-Apt.

FIG. 17 shows the results of Western blot analysis of HeLa cells treated and incubated with AuNP-GST-Apt-GST-FOXL2 to confirm GST-FOXL2 protein internalization mediated by AuNP-GST-Apt.

FIG. 18 shows the results of Western blot analysis of the nuclear extract to confirm nuclear localization of GST-FOXL2.

FIG. 19 shows the results of real time-PCR using cDNA library synthesized from RNAs isolated from the HeLa cells treated and incubated with AuNP-GST-Apt-GST-FOXL2.

FIG. 20 shows the results of confocal microscopy analysis of intracellular delivery of BIM protein into HeLa cells by a gold nanoparticle-aptamer conjugate (AuNP-His-Apt) of the present invention.

FIG. 21 shows the results of flow cytometry analysis of intracellular delivery of BIM protein into HeLa cells by the gold nanoparticle-aptamer conjugate (AuNP-His-Apt) of the present invention.

FIG. 22 shows the results of western blot analysis of intracellular delivery of BIM protein into HeLa cells by the gold nanoparticle-aptamer conjugate (AuNP-His-Apt) of the present invention.

FIG. 23 shows the results of flow cytometry analysis of intracellular delivery of BIM protein into HeLa cells by the gold nanoparticle-aptamer conjugate (AuNP-His-Apt) of the present invention.

FIG. 24 shows the results of cell viability after HeLa cells are treated with AuNP-His-Apt-His-BIM.

FIG. 25 shows the results of confocal microscopy analysis of intracellular delivery of BIM protein into human primary granulosa cells by the gold nanoparticle-aptamer conjugate (AuNP-His-Apt) of the present invention.

FIG. 26 shows the results of western blot analysis of intracellular delivery of BIM protein into human primary granulosa cells by the gold nanoparticle-aptamer conjugate (AuNP-His-Apt) of the present invention.

FIG. 27 shows the results of confocal microscopy analysis of intracellular delivery of BIM protein into KGN cells by the gold nanoparticle-aptamer conjugate (AuNP-His-Apt) of the present invention.

FIG. 28 shows the results of western blot analysis of intracellular delivery of BIM protein into KGN cells by the gold nanoparticle-aptamer conjugate (AuNP-His-Apt) of the present invention.

FIG. 29 shows the results of confocal microscopy analysis showing co-localization of AuNP-His-Apt (Red) and His-BIM (Green) in HeLa cells treated with Cy5-AuNP-His-Apt-His-BIM.

FIG. 30 shows the results of confocal microscopy analysis of endosomal marker proteins (early endosome antigen 1 (EEA), or lysosomal-associated membrane protein 1 (LAMP1)).

FIG. 31 shows the results of flow cytometry performed on the cells in FIG. 29 in the presence or absence of FBS.

FIG. 32 shows the transmission electron microscopy (TEM) images of the cross-section of the HeLa cells treated with AuNP-His-Apt-His-BIM.

FIG. 33 shows the results of quantitative analyses performed to evaluate the aptamer-loading capacity of a gold nanoparticle (AuNP).

FIG. 34 shows the protein-binding capabilities of AuNP-His-Apt or AuNP-GST-Apt.

FIG. 35 shows the measurements of cell viability in the HeLa cells treated and incubated with various concentrations of AuNP-His-Apt (0, 0.5, 1.0 and 2 nM).

FIG. 36 shows the results of confocal microscopy and western blot analyses showing that by AuNPs conjugated with both His-tag and GST-tag aptamers, each of the two proteins, His-BCL-xL and GST-FOXL2, was appropriately delivered into a corresponding intracellular compartment.

FIG. 37 shows the measurements of changes in tumor volume and weight over time after injection of an AuNP-His-Apt-His-BIM complex into an *in vivo* mouse xenograft model.

FIG. 38 shows the results of confocal microscopy analysis on His-BIM delivery to the tumor tissues by the AuNP-His-Apt conjugate in the *in vivo* mouse xenograft model.

FIG. 39 shows the results of Western blot analysis on His-BIM delivery to the tumor tissues by the AuNP-His-Apt conjugate in the *in vivo* mouse xenograft model.

FIG. 40 shows the results of immunohistochemical analysis on His-BIM delivery to the tumor tissues by the AuNP-His-Apt conjugate in the *in vivo* mouse xenograft model.

FIG. 41 shows the results of confocal microscopy analysis on targeted protein delivery capability of AuNP-His-Apt in the *in vivo* mouse xenograft model.

FIG. 42 shows the results of the relative fluorescence intensities of tumor and organ sections using Image J software, in order to confirm targeted protein delivery capability of AuNP-His-Apt in the *in vivo* mouse xenograft model.

FIG. 43 shows the results of immunohistochemical analysis or tumor using anti-BIM antibodies to confirm targeted protein delivery capability of AuNP-His-Apt in the *in vivo* mouse xenograft model.

FIG. 44 shows the results of confocal microscopy and western blot analyses of systemic distribution of His-BIM protein by AuNP-His-Apt in order to confirm biodistribution of an AuNP-His-Apt-Alexa488-His-BIM complex.

FIG. 45 shows the results of relative fluorescence intensities in the tissues by using Image J software to confirm biodistribution of the AuNP-His-Apt-Alexa488-His-BIM complex.

FIG. 46 shows changes in release levels of AST and LDH caused by AuNP-His-Apt-Alexa488-His-BIM, thereby confirming biodistribution of the AuNP-His-Apt-Alexa488-His-BIM complex.

[Modes of the Invention]

The inventors have completed the present invention as a result of research on a protein delivery carrier that has an excellent intracellular protein delivery capability and a very low cytotoxicity.

Hereinafter, the present invention will be described in further detail.

The present invention provides a protein delivery carrier including a gold nanoparticle and an aptamer conjugated to a surface of the gold nanoparticle, wherein the aptamer is selected from an aptamer that has SEQ ID NO: 1 and can specifically bind to a histidine-tag and/or an aptamer that has SEQ ID NO: 2 and can specifically bind to a glutathione S-transferase (GST)-tag.

The term "nanoparticle(s)" in the present invention is intended to mean particles of various materials having a diameter of nano unit. If the nanoparticle is particles having a nano size, it is not particularly limited In the present invention, the term "gold nanoparticle(s)" refers to a particle of gold metal having a diameter of nano unit. The small size of the particle allows penetration of nanoparticles of the present invention into the target cells (for example, human cells) as a result, making penetration of a protein delivery carrier into the cell interior possible.

Gold nanoparticles are relatively easy to prepare in a stable particle form, and also can be manufactured in various sizes from 0.8 nm to 200 nm suitable for its intended application. Gold can be structurally modified by combining with a variety of molecules such as peptides, proteins, nucleic acids and can reflect the light at various wavelengths, thereby being easily detectable inside cells. Further, unlike heavy metals such as manganese, aluminum, cadmium, lead, mercury, cobalt, nickel, beryllium, gold nanoparticles are harmless to the human body, having high biocompatibility and very low cytotoxicity.

Gold nanoparticles with diameter equal to or greater than 100 nm no longer exhibit nanomaterial characteristics, and the surface of gold without nanomaterial characteristics possesses weak binding ability to thiol groups and further, gold nanoparticles with diameter outside the range of 10 to 20 nm induce cytotoxicity. It is therefore preferable to maintain the size of the gold nanoparticle of the present invention within the range of 10 to 20 nm.

In the present invention, the term "aptamer" refers to a single-strand nucleic acid (DNA, RNA or modified nucleic acids) having a stable three-dimensional structure of itself, and high affinity to and selectivity for a target molecule. Aptamers binding to various target materials (protein, sugars, dyes, DNA, metal ions, cells, etc.) can be developed through Systematic Evolution of Ligands of Exponential enrichment (SELEX).

Also, for protein delivery carrier, aptamers to be conjugated with gold nanoparticles may be any aptamer of various tags, or a protein-specific aptamer of any kind, there is no particular limitation thereof.

Protein delivery carriers of the present invention can effectively deliver the target protein to the cell interior by specifically binding aptamers of various tags or protein-specific aptamers conjugated with gold nanoparticles with the target protein to be delivered, and therefore can be useful as a diagnostic or therapeutic tool in biomedical applications.

Disclosed is a complex formed by conjugation of the protein delivery carrier and a protein.

Also, disclosed is a method of delivering a protein including administering the above-described complex to an individual. The "individual" used herein means a subject needing to be treated or diagnosed, and more specifically, human or non-human primates, and mammals including mice, rats, dogs, cats, horses, and cows.

In an embodiment of the present invention, a protein delivery carrier was prepared by conjugating histidine-tagged aptamers or GST-tagged aptamers to gold nanoparticles (see Example 2), and the protein delivery carrier described above was found to be effective in intracellular delivery of proteins, namely, AcrA protein (Example 3), BCL-xL protein (Example 4), FOXL2 protein (Example 5), BIM protein (Example 7).

In another embodiment of the present invention, the aptamer-loading capacity of gold nanoparticles (AuNP) was evaluated and it was found that one AuNP can load 25 aptamers (See Example 9).

In still another embodiment of the present invention, it was found that the protein delivery carrier (gold nanoparticle-aptamer conjugate) of the present invention has no detectable cytotoxicity (See Example 10) and capable of delivering multiple proteins at once (See Example 11).

Furthermore, in yet another embodiment of the present invention in which *in vivo* antitumor activity (See Example 12) and biodistribution (See Example 14) were evaluated with respect to injection of AuNP-His-Apt-His-BIM, it was found that AuNP-His-Apt-His-BIM is effective at delivering His-BIM protein also *in vivo.*

Hereinafter, embodiments will be described in detail in order to improve the understanding of the present invention. However, the embodiments set forth are provided merely to improve the understanding of the present invention, and therefore are not to be interpreted as limiting the scope of the present invention.

**[Examples]**

**Example 1. Experimental Method**

1-1. Animal Preparation

6-week-old BALB/c nu/nu immunodeficient mice (Charles River Laboratories International, Inc., Japan) weighing from 18 to 20 g and 18-day-old Sprague-Dawley rats (Samtako, Korea) were injected subcutaneously with 2 mg of diethylstilbesterol (DES; Sigma) daily for three days. The animal room in which all mice were housed was maintained at a humidity of 30-40% and a temperature of $22 \pm 1°C$. The lighting in the room was on a 12-h light/dark cycle. Animal protocols were approved by the Chung-Ang University Support Center for Animal Experiments, and the animals were treated as described in the protocol.

1-2. Mammalian Cell Culture

HeLa (Human cervical carcinoma) cells were cultured in Dulbecco's modified Eagle's medium (DMEM). A431 (Human epidermoid carcinoma) cells were cultured in RPMI medium containing a 2.5 mM HEPES (N-(2-hydroxyethyl) piperazine-N'-2-ethanesulfonate) buffer. Human adult-type granulosa cell tumor-derived KGN and primary rat granulosa cells were cultured in Dulbecco's modified Eagle's medium/F12. All media contained 10% heat-inactivated fetal bovine serum (Caisson, USA) and 1% penicillin-streptomycin (Welgene, Korea).

1-3. Preparation of AuNP-Apt-protein complex

An AuNP-Apt conjugate was pre-incubated at 80°C for 5 minutes to prevent formation of secondary structures. AuNP-His/GST-Apt (1 nM) and purified His/GST-tagged protein were incubated at room temperature in 1x PBS containing 5 mM $MgCl_2$ (pH 7.2) for 10 minutes, the resulting conjugates were centrifuged at 13,000 $\times$ g, and the supernatant was removed. The AuNP-Apt-protein complex was washed with TBST supplemented with 500 mM NaCl and 1M KCl.

1-4. *In Vivo* Mouse Xenograft Model

HeLa cells ($1 \times 10^7$) were subcutaneously injected into 6-week old BALB/c-nu/nu mice (purchased from Central Lab Animal Inc, Korea) with weights ranging from 18 to 20g, and an AuNP-GST-Apt-His-BIM or AuNP-His-Apt-His-BIM complex was directly injected into tumor cells (tumor volume of approximately 0.1 cm$^3$). The long and short axial lengths of the tumors were measured every other day. Mice were killed 30 days after the first injection of the complex, and tumor samples were collected for further analyses. Results are expressed as the mean $\pm$ S.E.M. of the tumors of five mice in each group, and asterisks indicate statistically significant values compared to the corresponding controls (P < 0.05).

1-5. Recombinant Protein Purification

The Histidine-tagged proteins were produced in BL21(DE3) *E. coli* cells and were purified using Ni-NTA agarose resin (Qiagen, USA) or GST Sepharose 4B beads (GE Life science, USA). His- or GST-tags were removed from purified proteins using the TAGZyme System (Qiagen) and Thrombin (GE Life science). For the protein uptake experiments, proteins were labeled with the Alexa Fluor® 488 Microscale Protein Labeling Kit (Invitrogen, USA) or the Texas Red® X Protein Labeling Kit (Invitrogen).

1-6. Analysis on Protein-Delivery Capabilities of Gold Nanoparticle-Aptamer Conjugate using Confocal Microscopy

To detect the delivery of the proteins into the cells, cells grown on 10 mm lysine-coated cover slips were incubated with the AuNP-Apt-protein complex for 1 hour, and then fixed with 4% paraformaldehyde (Sigma, USA). Alexa488 (495 nm excitation, 519 nm emission), Alexa546 (556 nm excitation, 573 nm emission), and Cy5 (646 nm excitation, 670 nm emission) fluorescence were detected by laser scanning confocal microscopy (Carl Zeiss ZEN 2011, Germany). The relative fluorescence intensities were measured using Image J software (NIH, USA).

1-7. Cell Viability Assay

HeLa cells were seeded in 96-well plates and cultured overnight to allow for cell attachment. The cells were incubated with AuNP-His-Apt-His-BIM, and cell viability was measured (Yeom, J.H. et al. Inhibition of Xenograft Tumor Growth by Gold Nanoparticle-DNA Oligonucleotide Conjugates-Assisted Delivery of BAX mRNA. PLoS One 8, e75369 (2013)).

1-8. Subcellular Fractionation

HeLa cells were treated with AuNP-His-Apt-His-BIM and incubated for 1 hour, and then the mitochondrial fractions were collected using a mitochondria isolation kit (Thermo, USA).

HeLa cells were treated with AuNP-GST-Apt-GST-FOXL2 and incubated for 1 hour, and then the nuclear fraction was isolated using NE-PER® nuclear and cytoplasmic extraction reagents (Thermo).

### 1-9. Reverse Transcription and Real-Time PCR Analysis

Reverse transcription and real-time PCR were performed by the method previously disclosed (Kim, J.H. et al. Differential apoptotic activities of wild-type FOXL2 and the adult-type granulosa cell tumor-associated mutant FOXL2 (C134W). Oncogene 30, 1653-1663 (2010)).

### 1-10. Immunohistochemical Analysis

Tumor sections were prepared and immunostained (Biomaterials). Polyclonal anti-BIM antibody (1:100) (Santa Cruz Biotechnology, USA) diluted in the antibody diluent (Dako, USA) was used.

### 1-11. Statistical Analysis

Multiple comparison analyses of values were performed by the Student-Newman-Keuls test (SAS, USA) and the significance of values was analyzed by comparison to controls with Student's t-test (SAS). Data represent the mean $\pm$ SEM, and $P < 0.05$ was considered to be statistically significant. The P values of significant results are shown.

### Example 2. Preparation of Protein Delivery Carrier of Gold Nanoparticle-Aptamer Conjugate

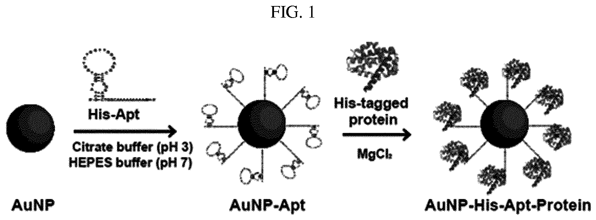

A protein delivery carrier was prepared by using a his-tag aptamer and gluthathione S-transferase (GST), as illustrated in the schematic diagram in FIG. 1.

#### 2-1. Preparation of protein delivery carrier (AuNP-His-Apt) using his-tag aptamer

A) Pretreatment of DNA Aptamer

To prepare the protein delivery carrier of the present invention, a DNA aptamer (his-tag aptamer) that specifically detects and binds to histidine was used. More specifically, the histidine-tag DNA aptamer is a 3'-thiol modified aptamer, whose sequence consists of 5'-GCTATGGGTGGTCTGGTTGGGATTGGCCCCGGGAGCTGGCAAAAAAAAAA-3'(SH) (SEQ ID NO: 1). The dried DNA aptamer was dissolved in water to reach a final concentration of 100 $\mu$M and reacted with 50 $\mu$l of an oligonucleotides to which 1N DTT (dithiothreitol) was added for 15 minutes at room temperature. In order to remove the DTT with unwanted thiol groups, the DTT was mixed with 50 $\mu$l of ethylacetate, and the supernatant was removed by centrifugation. The process was repeated 3 times. Aptamers were precipitated by ethanol (EtOH) precipitation method.

B) Conjugation of Gold Nanoparticle and DNA Aptamer.

The precipitated DNA aptamers obtained by pretreatment from Example 2-1 A) was dissolved in water and added to gold nanoparticles. For gold nanoparticles (AuNP), a gold colloid-15nm (#EM.GC15) purchased from BBI Life Science (UK) was used. Also, AuNPs were conjugated with a 5'-Cy5-labeled His-aptamer (Bioneer) in order to detect localization of the AuNPs.

2 nM AuNP was sufficiently mixed with the aptamers (DNA : AuNP = 100 : 1), and then a citrate buffer (0.5 M, pH 3) was added to the mixture such that the final concentration was 10 mM. The resultant mixture was then allowed to react for 3 to 5 minutes at room temperature and after the reaction was completed, in order to neutralize the gold, a HEPES buffer (0.5 M, pH 7.6) was added such that the final concentration was 30 mM and allowed to react for 10 minutes at room temperature. The mixture of aptamers and gold was then collected by centrifugation at ~10,000 x g for 20 minutes and the unreacted oligo in the supernatant was removed. This process was repeated 3 times. The final aptamer-gold conjugate was dispersed into HEPES buffer (5 mM, pH 7.6).

#### 2-2. Preparation of Protein Delivery Carrier (AuNP-GST-Apt) by using GST-tag Aptamer

A) Pretreatment of DNA aptamer

The protein delivery carrier according to the present invention was prepared by using a DNA aptamer (GST-tag aptamer) that detects and specifically binds to glutathione S-transferase (GST). More specifically, the GST-tag DNA aptamer is an aptamer with 3'-end thiol-modified whose sequence consists of 5'-CTGCCCCGCTATAGAACACCCGTTGGGCAAATGTGTTCGAAAAAAAAAAA-3'(SH) (SEQ ID NO: 2). The GST-tag DNA aptamer was pretreated by the method described in Example 2-1 A).

B) Conjugation of Gold Nanoparticle and DNA Aptamer

Pretreated and precipitated DNA aptamers from Example 2-2 A) were conjugated with gold nanoparticles by the method described in Example 2-1 B) to prepare the final aptamer-gold conjugate.

### Example 3. Experiments on Intracellular Delivery Capability of *E. coli* AcrA protein by Gold Nanoparticle-Aptamer Conjugate

#### 3-1. Confocal Microscopy Analysis

1 nM gold nanoparticle-aptamer conjugate prepared from Example 2-1 was subjected to 80 °C for 5 minutes and cooled down to room temperature. The conjugate was then allowed to react with 1-2 $\mu$g/$\mu$l histidine-tagged AcrA (His-AcrA) or untagged AcrA protein (AcrA)

in 5 mM MgCl$_2$-PBS buffer at room temperature for 2 hours. The reacted gold nanoparticle-aptamer-protein complex was then centrifuged at ~10,000 x g for 20 minutes and the supernatant was removed.

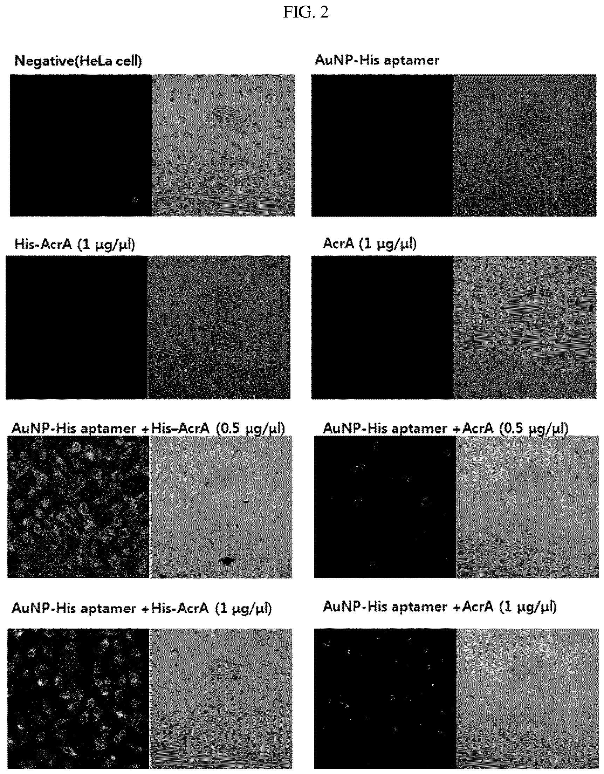

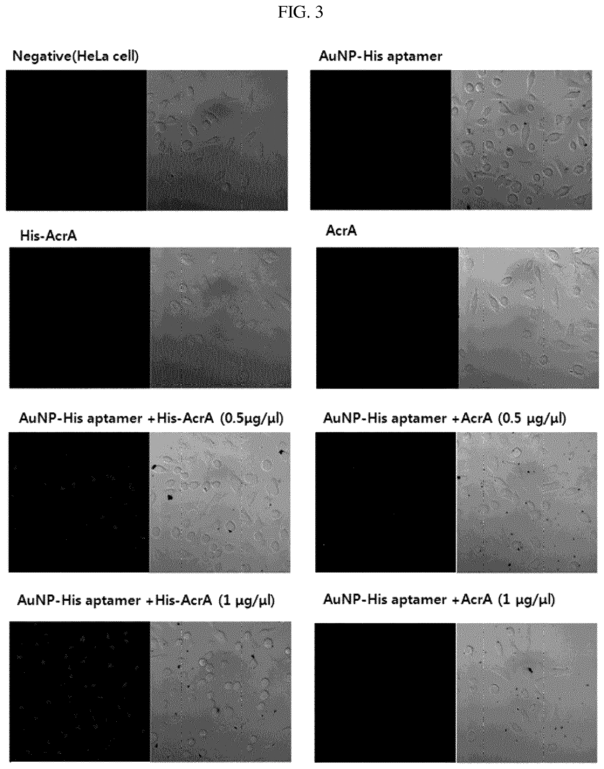

Human cervical cancer cells (HeLa cells) were treated with the gold nanoparticle-aptamer-protein complex and incubated for 1 hour. After the incubation, the cells were washed with PBS and fixed with 4% paraformaldehyde. The fixed samples were immunostained and observed using confocal microscopy. The results are shown in FIG. 2 and 3.

More specifically, FIG. 2 shows the results of confocal microscopy analysis for *E.coli* AcrA protein delivery using secondary antibodies labeled with Rabbit-488 (Green), and FIG. 3 shows the results of confocal microscopy analysis for *E.coli* AcrA protein delivery using secondary antibodies labeled with Rabbit-584 (Red). Because the histidine-tag aptamer of the histidine-tag aptamer-gold nanoparticle conjugate detects and specifically binds to histidine, as shown in FIG. 2 and FIG. 3, the histidine-tag aptamer-gold nanoparticle conjugate conjugated with histidine-tagged AcrA proteins showed greater intracellular protein delivery capability than those conjugated with untagged AcrA proteins.

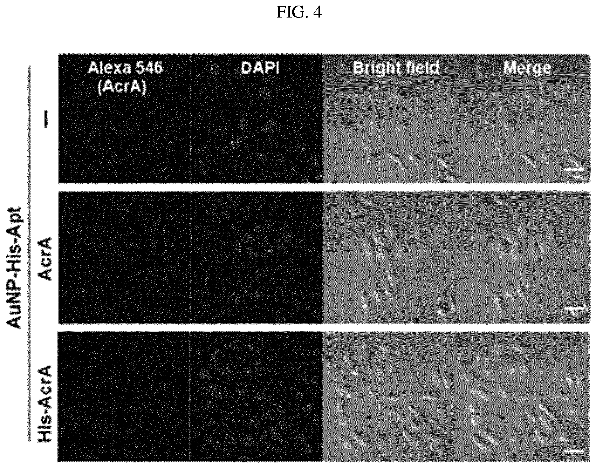

Further, HeLa cells treated with the AcrA or His-AcrA proteins that are conjugated or unconjugated with AuNP-His-Apt, were incubated for 1 hour and immunostained using anti-AcrA antibodies and Alexa 546 rabbit-IgG antibodies, and then analyzed using confocal fluorescence microscopy. The results are shown in FIG. 4. Representative confocal fluorescence images were observed with a 40× water-immersion objective using excitation at 556 nm and emission at 573 nm.

### 3-2. Western Blot Analysis

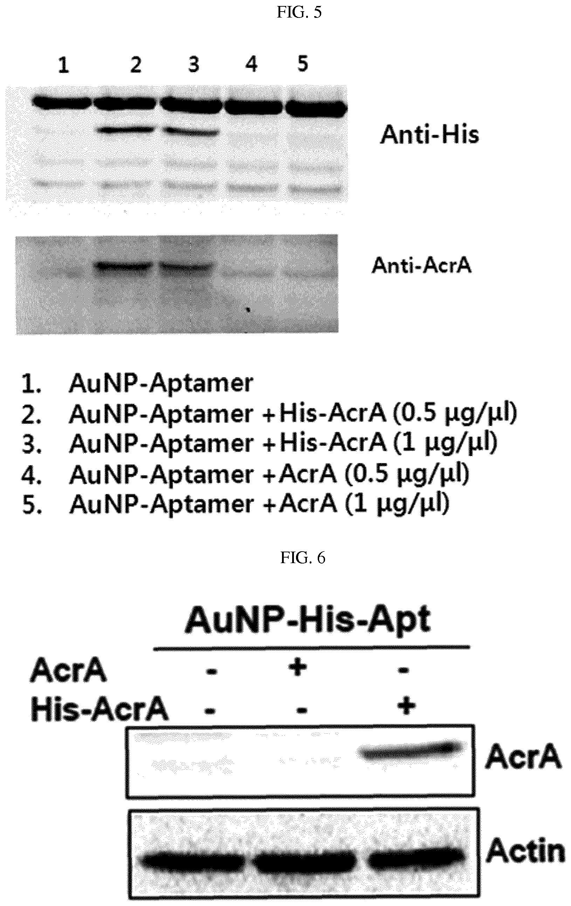

To detect proteins delivered into the cells, a Western blot analysis was performed and the results are shown in FIG. 5.

As shown in FIG. 5, AcrA proteins were detected when the histidine-tag aptamer-gold nanoparticle conjugate was conjugated with histidine-tagged AcrA protein (lane 2 and lane 3).

Further, HeLa cells treated with the AcrA or His-AcrA proteins that are conjugated or unconjugated with AuNP-His-Apt, were incubated for 1 hour and the Western blot analysis was performed using anti-AcrA antibodies. The results are shown in FIG. 6.

### Example 4. Experiments on Intracellular Delivery Capability of human BCL-XI protein by Gold Nanoparticle-Aptamer Conjugate

#### 4-1. Confocal Microscopy Analysis

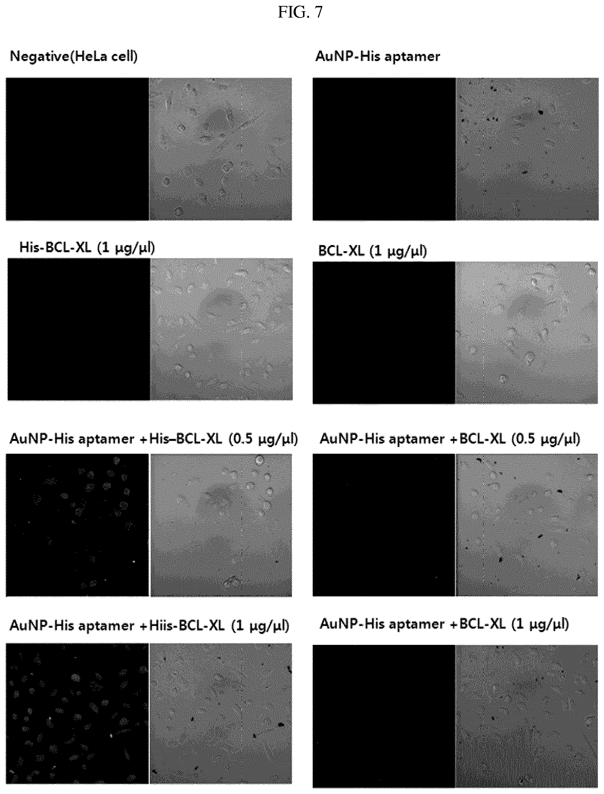

Intracellular delivery capability of BCL-xL protein was confirmed by the method described in Example 3-1, and the results are shown in FIG. 7.

More specifically, FIG. 7 shows the results of confocal microscopy analysis of delivery of the BCL-xL protein by using Rabbit488 (Green)-labeled secondary antibodies, and as shown in FIG. 7, the histidine-tag aptamer-gold nanoparticle conjugate conjugated with histidine-tagged BCL-xL proteins was shown to have greater intracellular protein delivery capability than those conjugated with untagged BCL-xL proteins.

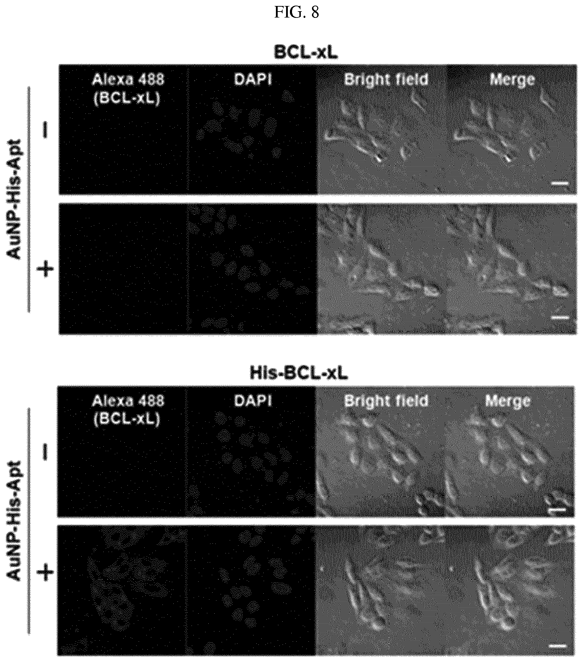

Further, HeLa cells treated with the BCL-xL or His-BCL-xL proteins that are conjugated or not conjugated with AuNP-His-Apt, were incubated for 1 hour and immunostained using anti-BCL-xL and Alexa 488-labeled mouse-IgG, and then analyzed using a confocal fluorescence microscope. The results are shown in FIG. 8. Representative confocal fluorescence images were observed with a 40× water-immersion objective and the scale bar was 20 μm.

#### 4-2. Western Blot Analysis

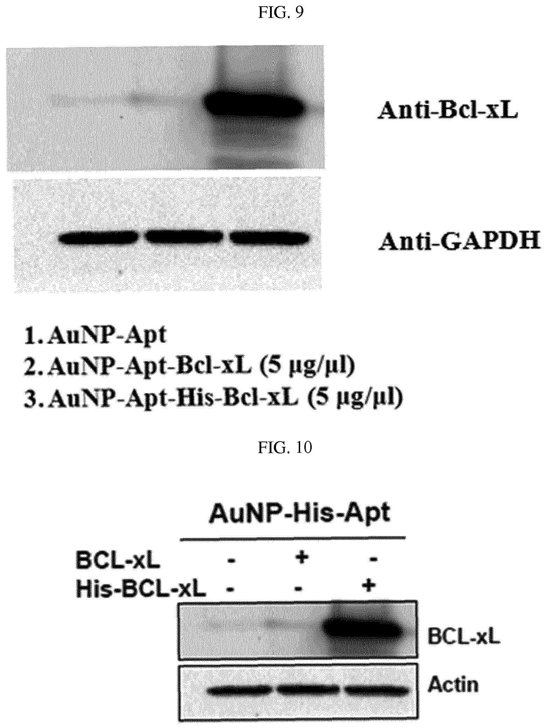

By the method described in Example 3-2, intracellular delivery of BCL-xL protein was confirmed by Western blotting and the results are shown in FIG. 9.

More specifically, FIG. 9 shows the results of Western blot analysis performed to detect BCL-xL protein, and as shown in FIG. 9, when histidine-tag aptamer-gold nanoparticle conjugates were conjugated with histidine-tagged BCL-xL proteins (lane 3), the BCL-xL protein was detected.

Further, HeLa cells treated with BCL-xL or His-BCL-xL proteins that are conjugated or unconjugated with AuNP-His-Apt, were incubated for 1 hour and analyzed by Western blotting. The results are shown in FIG. 10.

#### 4-3. Flow Cytometry Analysis

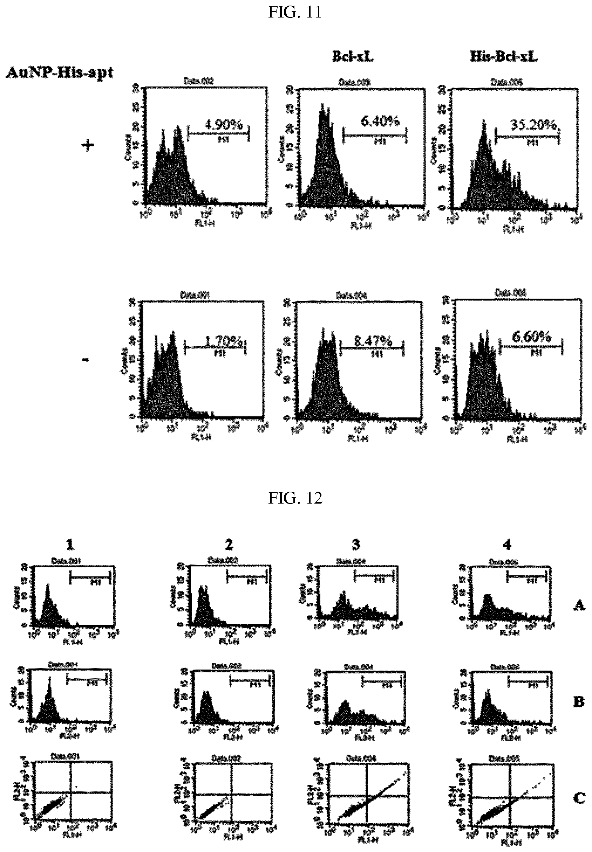

To further confirm intracellular delivery capability by the His-tag aptamer gold nanoparticle conjugate prepared in Example 2-1, the complex was prepared by the method described in Example 3-1 using Alexa488-His-BCL-xL protein and Alexa488-BCL-xL protein. Once the complex is prepared, the supernatant was removed and HeLa cells were treated with the resultant aptamer-gold nanoparticle-protein complex and incubated for 1 hour. After the incubation, the cells were washed with PBS and dispersed into 1 mL of PBS buffer. The samples were observed by flow cytometry and the results are shown in FIG. 11 and Fig 12.

More specifically, FIG. 11 shows the results of flow cytometry analysis of protein delivery of Alexa488 (Green) fluorophore-labeled His-BCL-xL protein and BCL-xL protein. FIG. 12 shows the flow cytometry results that show the number of cells having both red wavelength from the gold nanoparticle and green wavelength from the protein using BCL-xL protein complex prepared by combining a Cy5 (Red)-labeled gold nanoparticle-aptamer conjugate and Alexa488 (Green) fluorophore-labeled His-BCL-xL protein.

As shown in FIG. 11, the His-tag aptamer-gold nanoparticle conjugate conjugated with His-tagged BCL-xL protein showed higher intracellular protein delivery capability than that of the conjugate conjugated with untagged BCL-xL protein.

Also as shown in FIG. 12, it was confirmed that the His-tag aptamer-gold nanoparticle conjugate are present along with His-tagged BCL-xL protein inside the cells.

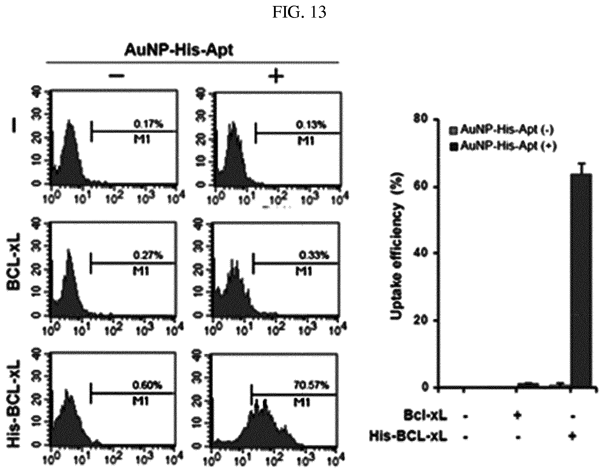

Further, HeLa cells treated with BCL-xL or His-BCL-xL proteins that were conjugated or not conjugated with AuNP-His-Apt, were incubated for 1 hour and flow cytometry analysis was performed on Alexa 488-positive cells. The results are shown in FIG. 13.

## Example 5. Experiments on Intracellular Delivery Capability of human FOXL2 protein by Gold Nanoparticle-Aptamer Conjugate

### 5-1. Confocal Microscopy Analysis

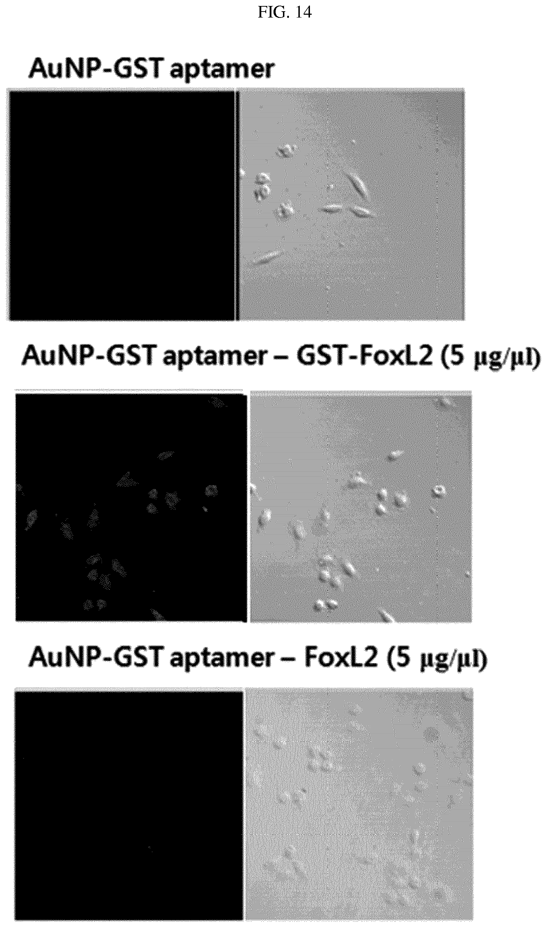

Intracellular delivery capability of GST-FOXL2 protein was confirmed by the method described in Example 3-1 using the gold nanoparticle-aptamer conjugate prepared in Example 2-2. The results are shown in FIG. 14.

More specifically, FIG. 14 shows the results of confocal microscopy analysis of protein delivery of FOXL2 using Rabbit-Rodamine (Red)-labeled secondary antibodies. As shown in FIG. 14, the GST-tag aptamer-gold nanoparticle conjugate conjugated with GST-tagged FOXL2 proteins was shown to have greater intracellular protein delivery capability than those conjugated with untagged FOXL2 proteins.

### 5-2. Western Blot Analysis

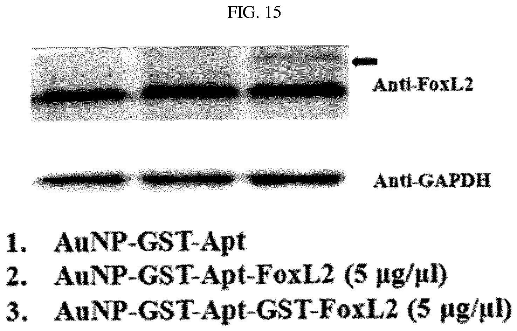

Intracellular delivery capability of GST-FOXL2 protein was confirmed by Western blotting as described in Example 3-2 using the gold nanoparticle-aptamer conjugate prepared in Example 2-2. The results are shown in FIG. 15.

More specifically, FIG. 15 shows the results of Western blot analysis for FOXL2 protein detection, and as is shown, FOXL2 protein was detected when the GST-tag aptamer-gold nanoparticle conjugate was conjugated with GST-tagged FOXL2 protein (lane 3).

## Example 6. Confirmation of Internalization of GST-FOXL2 protein by AuNP-GST-Apt

### 6-1. Confocal Microscopy Analysis

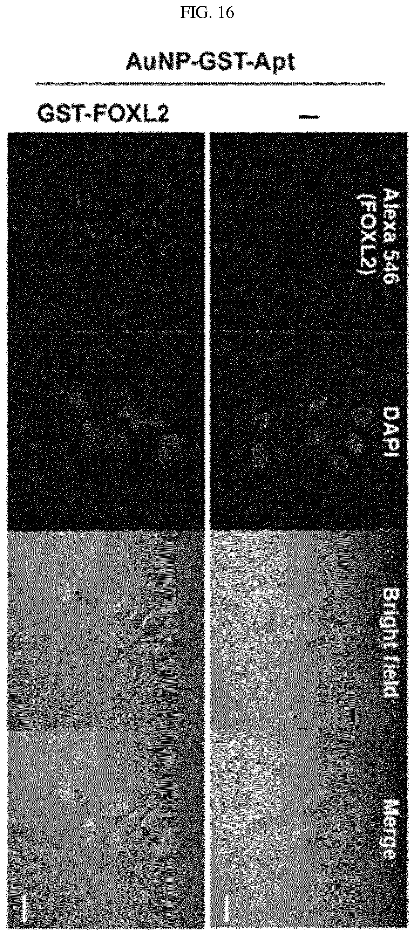

An aptamer-gold nanoparticle-protein complex (AuNP-GST-Apt-GST-FOXL2) was prepared by reacting GST-tagged FOXL2 protein (GST-FOXL2) and AuNP-GST-Apt. HeLa cells treated with the above complex were incubated for 1 hour and immunostained using anti-FOXL2 and Alexa546-rabbit IgG. The cells were observed using confocal microscopy and the results are shown in FIG. 16. Images were observed with a 40× water-immersion objective using excitation at 556 nm and emission at 573 nm. The scale bar was 20 μm.

As shown in FIG. 16, it was confirmed that FOXL2 proteins were effectively delivered into HeLa cells. Also, it was observed that the FOXL2 proteins were localized in cell nuclei.

### 6-2. Western Blot Analysis

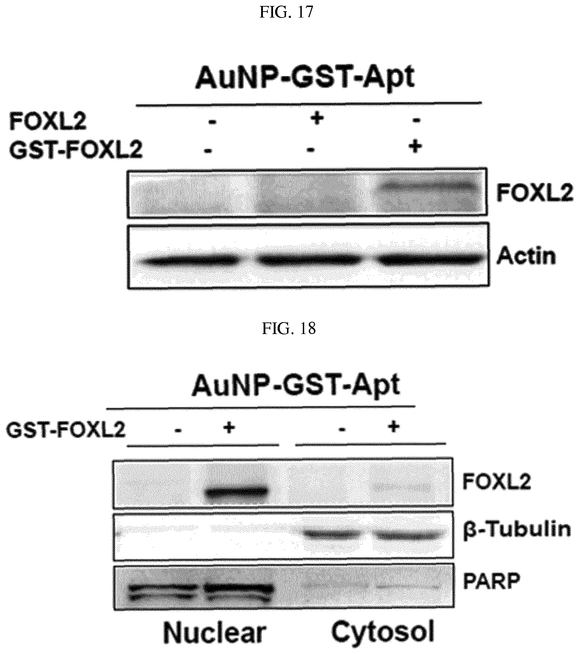

HeLa cells treated with AuNP-GST-Apt-GST-FOXL2 were incubated for 1 hour and analyzed by Western blotting, and the results are shown in FIG. 17.

As shown in FIG. 17, it was confirmed that FOXL2 proteins are effectively delivered into the HeLa cells.

Further, in order to verify GST-FOXL2 nuclear localization, western blotting was performed on the nuclear extract and the results are shown in FIG. 18. Here the nucleic and cytosolic fractions were isolated from the cells treated and incubated with AuNP-GST-Apt-GST-FOXL2 for 1 hour and these fractions were analyzed by Western blotting using appropriate antibodies. To verify the relative nuclear purification, all fractions were also western blotted with antibodies to detect nuclear (PARP) and cytoplasmic markers ($\beta$-tubulin).

As shown in FIG. 18, it was confirmed that FOXL2 proteins were localized in the cell nuclei.

### 6-3. Real-time PCR Analysis

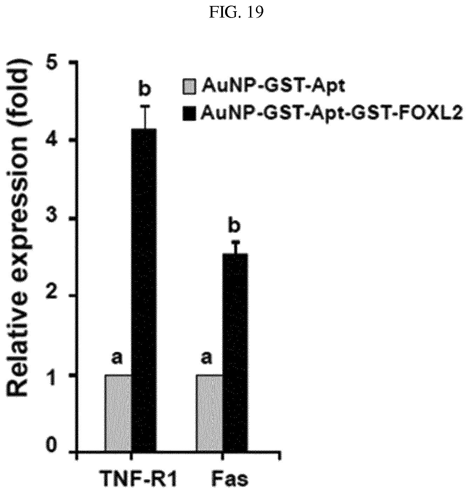

Real time PCR was performed using a cDNA library generated from RNAs isolated from HeLa cells treated and incubated with AuNP-GST-Apt or AuNP-GST-Apt-GST-FOXL2 for 1 hour, and the results are shown in FIG. 19. The results indicate the relative expression fold after normalization to glyceraldehyde 3-phosphate dehydrogenase (GAPDH) expression, and the above results (means $\pm$ SEM) were are from at least three independent experiments performed in triplicate. Statistically significant values are indicated as $P < 0.05$.

As shown in FIG. 19, it was confirmed that intracellular-delivered FOXL2 proteins effectively up-regulated target genes including TNF-R1 (tumor necrosis factor-receptor 1) and Fas (CD95/APO-1).

## Example 7. Experiments on Intracellular Delivery Capability of BIM protein by Gold Nanoparticle-Aptamer Conjugate

The following experiments were performed in order to verify the intracellular delivery of BIM proteins by the gold nanoparticle-aptamer conjugate (AuNP-His-Apt) of the present invention.

7-1. Intracellular BIM protein Delivery Capability into HeLa cell

A. Confocal Microscopy Analysis

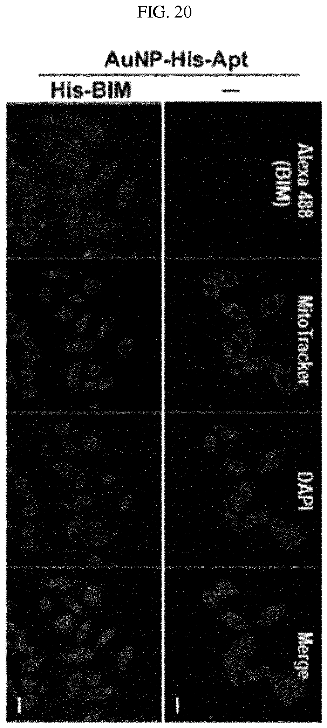

The aptamer-gold nanoparticle-protein complex (AuNP-His-Apt-His-BIM) was prepared by adding histidine-tags to AuNP-His-Apt, which then were reacted with Alexa 488 labeled BIM protein (His-BIM). Human cervical cancer cells (HeLa cells) were treated with the aptamer-gold nanoparticle-protein complex (AuNP-His-Apt-His-BIM), incubated for 1 hour and stained using Mitotracker (red) and DAPI, and then analyzed using a confocal microscope. The results are shown in FIG. 20. Here the final concentration of AuNP-His-Apt and His-BIM were respectively 1 nM and 0.4 $\mu$M and the cells were visualized using the 40x water immersion objective lens. The scale bar was 20 $\mu$m.

As shown in FIG. 20, the HeLa cells treated and incubated with AuNP-His-Apt loaded with Alexa 488-labeled His-BIM protein (Alexa 488-His-BIM) show green fluorescence of Alexa 488, whereas the cells treated with unloaded AuNP-His-Apt show no fluorescent signal. Also, it was observed that His-BIM proteins delivered by AuNP-His-Apt were localized predominantly in mitochondria. The above results show that intracellular-delivery of His-BIM proteins was done by the aptamer-gold nanoparticle conjugate of the present invention.

B. Flow Cytometry

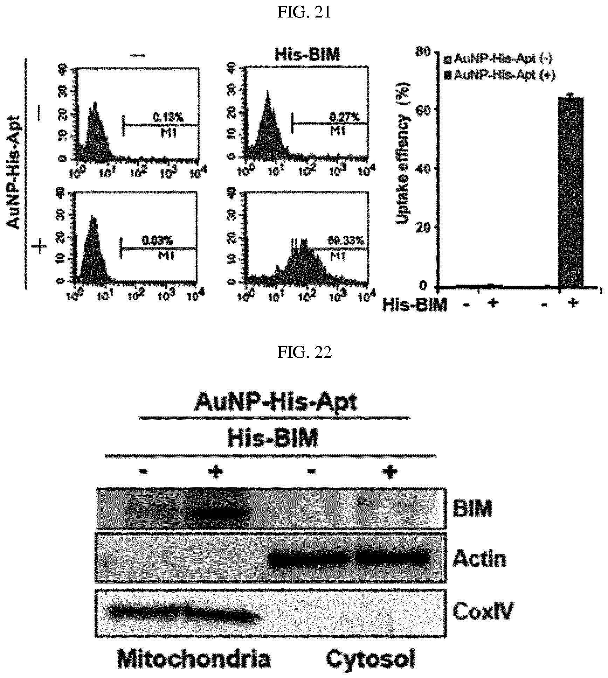

HeLa cells treated with Alexa 488-His-BIM proteins that are reacted or unreacted with AuNP-His-Apt were incubated for 1 hour, and observed by flow cytometry. The results are shown in FIG. 21.

As shown in FIG. 21, internalization of His-BIM proteins by AuNP-His-Apt was confirmed by flow cytometry analysis that identifies Alexa 488-positive cells. Intracellular-delivery of His-BIM proteins was found to be greater when AuNP-His-Apt was reacted with Alexa 488-His-BIM.

His-BIM protein uptake efficiency was obtained by repeating the experiment 3 times and results are presented as means ± SEM, in FIG. 21 (right panel).

As shown in FIG. 21, it was seen that His-BIM protein uptake efficiency was greater when AuNP-His-Apt was reacted with Alexa 488-His-BIM.

C. Western Blot Analysis

HeLa cells treated with the aptamer-gold nanoparticle-protein complex (AuNP-His-Apt-His-BIM) prepared by conjugating AuNP-His-Apt with His-BIM protein were incubated for 1 hour and analyzed by western blotting. The results are shown in FIG. 22. Efficient isolation of the mitochondrial and cytosolic fractions was confirmed by western blotting using anti-Cox IV and anti-$\beta$-actin.

As shown in FIG. 22, it was confirmed that His-BIM proteins were delivered into the cells by AuNP-His-Apt and particularly, localized predominantly in mitochondria.

D. Flow Cytometry Analysis

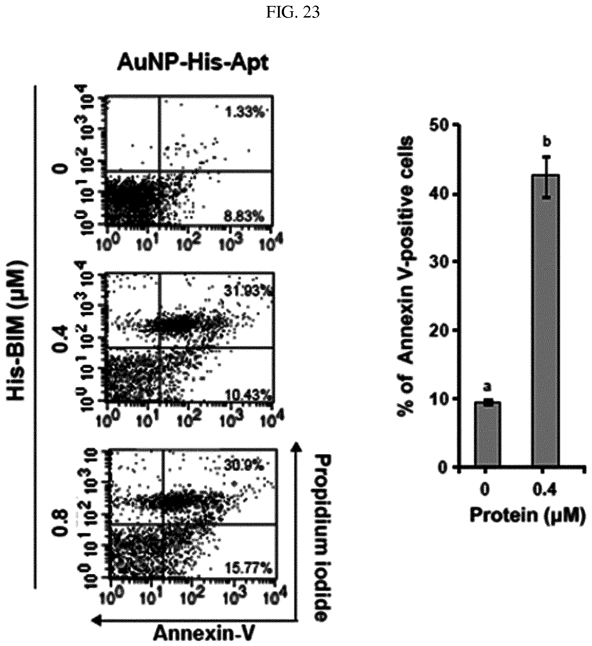

Cellular apoptosis induced by His-BIM protein was quantified by flow cytometry of Annexin V-positive apoptotic cells. That is, HeLa cells treated with the aptamer-gold nanoparticle-protein complex (AuNP-His-Apt-His-BIM) prepared by conjugating AuNP-His-Apt with His-BIM protein at predetermined concentrations (0.4 $\mu$M and 0.8 $\mu$M) were incubated for 12 hour and analyzed by flow cytometry, and the results are shown in FIG. 23. The results (means ± SEM) were obtained from three independent experiments, and unless otherwise defined, statistically significant values are indicated as P < 0.05.

As shown in FIG. 23, the Annexin V-positive apoptotic cells were found to be increasing in the cells treated with AuNP-His-Apt-His-BIM than the control group. From these results, it was confirmed that His-BIM proteins were delivered into the cell by the aptamer-gold nanoparticle conjugate of the present invention.

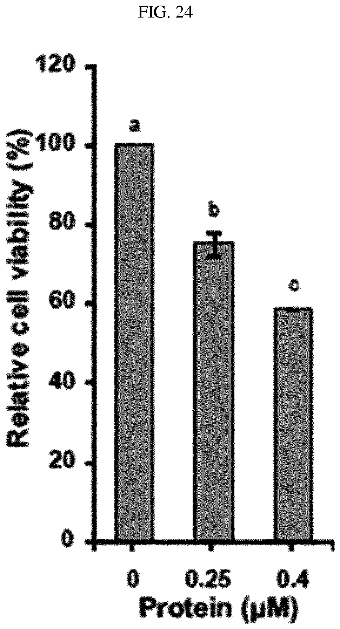

Meanwhile, localization of His-BIM protein can be controlled by regulating apoptosis-inducing factors such as cytochrome c, Smac/DIABLO and caspases activities in mitochondria. In addition, HeLa cells were treated with AuNP-His-Apt-His-BIM for 12 hours, and then cell viability was measured. The results are shown in FIG. 24. The results (means ± SEM) were obtained from experiments repeated 3 times.

As shown in FIG. 24, it was found that cell viability of the HeLa cells decreased in a manner dependent on the concentration of His-BIM protein delivered by AuNP-His-Apt conjugate.

7-2. Confirmation of Intracellular BIM protein Delivery to Human primary granulosa cells

A. Confocal Microscopy Analysis

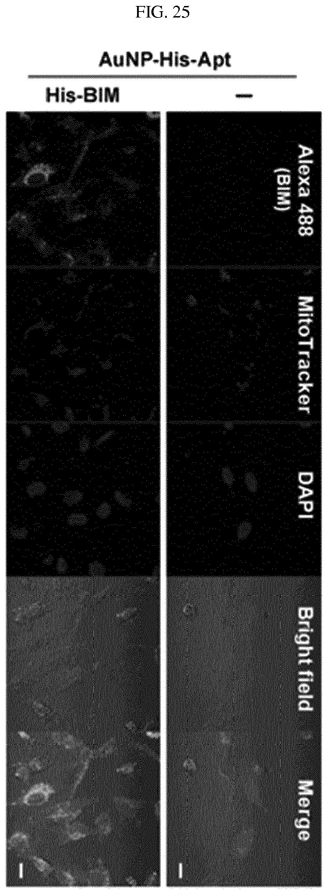

Human primary granulosa cells treated with AuNP-His-Apt (1 nM) or an AuNP-His-Apt-Alexa488-His-BIM complex were incubated for 1 hour and observed using confocal fluorescence microscope, and the results are shown in FIG. 25. Here, the cells were imaged using the 40x water immersion objective lens, and the scale bar was 20 $\mu$m.

B. Western Blot Analysis

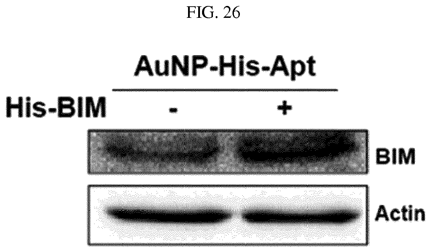

Human primary granulosa cells treated with AuNP-His-Apt (1 nM) or the AuNP-His-Apt-Alexa488-His-BIM complex were incubated for 1 hour and analyzed by western blotting, and the results are shown in FIG. 26.

7-3. Intracellular BIM protein Delivery Capability into KGN cells

A. Confocal Microscopy Analysis

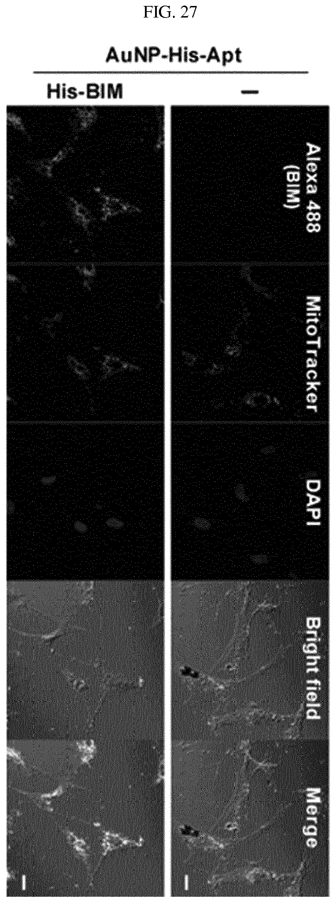

KGN cells treated with AuNP-His-Apt (1 nM) or an AuNP-His-Apt-Alexa488-His-BIM complex were incubated for 1 hour and observed using a confocal fluorescence microscope, and the results are shown in FIG. 27. Here the cells were imaged by using the 40x water immersion objective lens, and the scale bar was 20 μm.

B. Western Blot Analysis

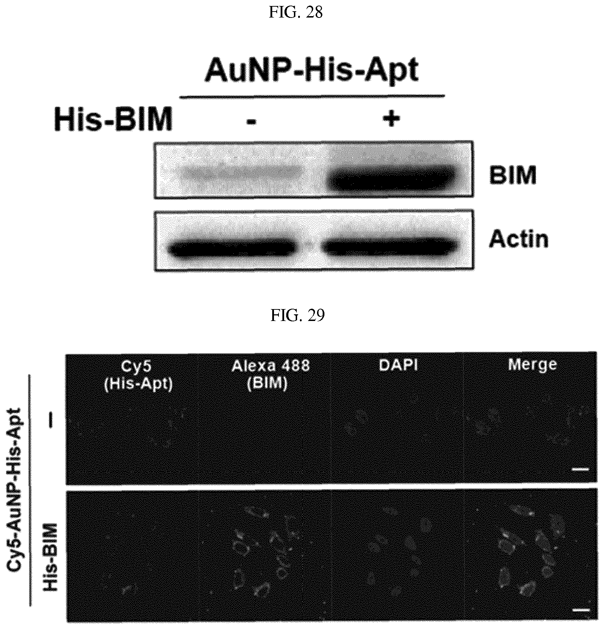

KGN cells treated with either AuNP-His-Apt (1 nM) or the AuNP-His-Apt-Alexa488-His-BIM complex were incubated for 1 hour and analyzed by western blotting, and the results are shown in FIG. 28.

**Example 8. Confirmation of Internalization of His-BIM protein by AuNP-GST-Apt**

HeLa cells treated with an aptamer-gold nanoparticle conjugate (Cy5-AuNP-His-Apt) whose 5'-end is labeled with fluorophore Cy5 (Cyanine 5) and an aptamer-gold nanoparticle-protein complex (Cy5-AuNP-His-Apt-His-BIM) prepared by reacting the aptamer-gold nanoparticle conjugate (Cy5-AuNP-His-Apt) and the BIM protein (His-BIM) labeled Alexa 488 were incubated for 1 hour, and then analyzed by confocal microscopy. The results are shown in FIG. 29. Here, the cells were imaged using the 40x water immersion objective and the scale bar was 20 μm.

As shown in FIG. 29, it was found that AuNP-His-Apt (Red) and His-BIM (Green) were co-localized in the cells.

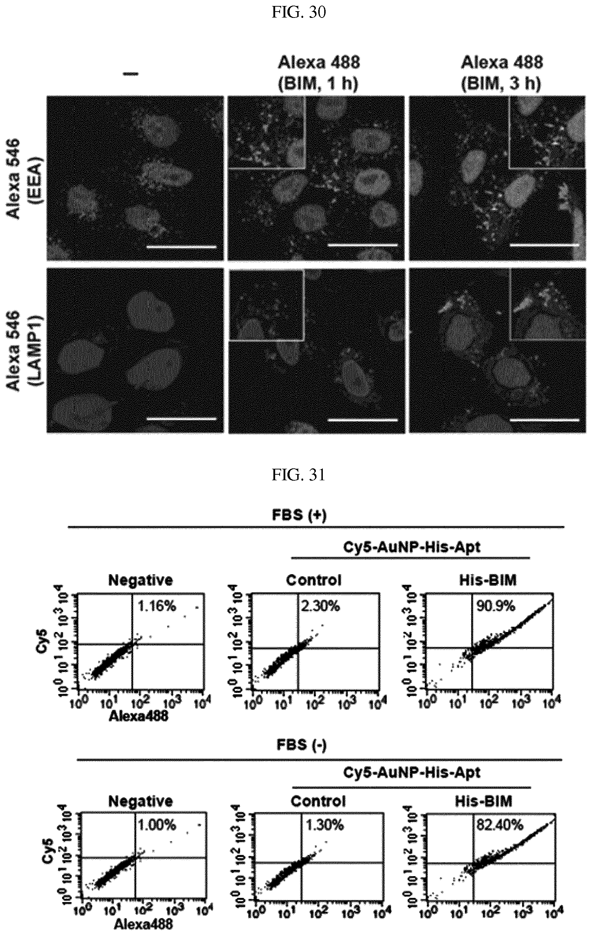

Further, confocal microscopy analysis was performed on endosomal marker proteins (early endosome antigen 1 (EEA), or lysosomal-associated membrane protein 1 (LAMP1)), and the results are shown in FIG. 30. More specifically, HeLa cells treated with AuNP-His-Apt-Alexa 488-His-BIM were incubated for 1 hour or 3 hours, and allowed to react with anti-EEA-1 (1:500) and anti-LAMPI (1:100) and analyzed by a confocal fluorescence microscope. Here, the cells were counterstained using Alexa Fluor 546 goat anti-mouse IgG (1:1,000) and Alexa Fluor 546 goat anti-rabbit IgG (1:1,000).

Also, flow cytometry analysis was performed on the cells in the presence or absence of FBS, and the results are shown in FIG. 31. The percentages in FIG. 31 represents is the positive cell groups for both Cy5 and Alexa 488.

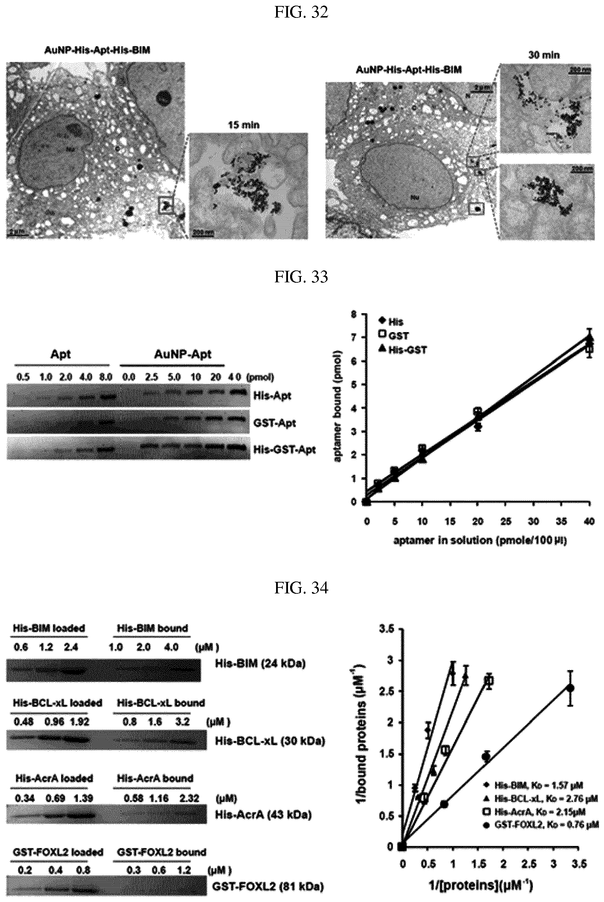

Also, HeLa cells (1 x 10[7]) treated with AuNP-His-Apt-His-BIM were incubated for 15 and 30 minutes respectively, and trypsinized, centrifuged, and then washed with PBS (phosphate buffered saline). Once trypsin was removed, cell pellets were fixed in the Karnovsky's glutaraldehyde-paraformaldehyde mixture in a 0.2 M cacodylate buffer (pH 7.4) at room temperature for 3 hours, and washed with the cacodylate buffer (pH 7.4) to remove the fixatives. The pellets were then dehydrated in an alcohol series and embedded in Spurr's resin, and sliced to a thick of 70 nm, and then observed by TEM (transmission electron microscopy). The results are shown in FIG. 32. Here, TEM images were taken with a JEOL model JEM-1010 system operated at 80 kV accelerating voltage at 10K and 25K magnifications.

From these results, it was confirmed that His-tagged BIM proteins were delivered by the AuNP-Apt conjugate.

**Example 9. Evaluation of Aptamer Loading Capacity of Gold nanoparticle (AuNP)**

Quantitative analysis of aptamers was performed to evaluate loading capacity of AuNP for His-apt or GST-apt.

More specifically, known amounts of GST-Apt, His-Apt, and His-GST-Apt (0, 0.5, 1, 2, 4, and 8 pM) were electrophoresed on an agarose gel and quantified to serve as standards. The pellet from the reaction solution of varying amounts (0, 2.5, 5, 10, 20, and 40 pmol) of these aptamers hybridized to AuNP (1 nM) were also quantified. Data are obtained from two independent experiments and the results are shown in FIG. 33.

Based on the calculation with quantitative data (right panel) and molecular weight, it was confirmed that about 25 aptamers were loaded on one AuNP.

Additionally, protein binding capacity of AuNP-His-Apt or AuNP-GST-Apt was measured. More specifically, known amounts of His-BIM, His-BCL-xL, His-AcrA, or GST-FOXL2 were electrophoresed on SDS-PAGE to serve as standards.

AuNP-apt (1 nM) was reacted with the indicated concentrations of His-BIM, His-BCL-xL, His-AcrA, or GST-FOXL2 in 10 μl of binding buffer and analyzed by SDS-PAGE. The amounts of proteins bound to AuNPs were determined by measuring the intensities of bands and comparing them with the standards. The results are shown in FIG. 34. The dissociation constants ($K_D$) were obtained using the slopes calculated from the graphs in the right panel.

As shown in FIG. 34, it was confirmed that 60% of the proteins were able to conjugate with one AuNP-His-Apt.

**Example 10. Cell Viability Assay of AuNP-His-Apt**

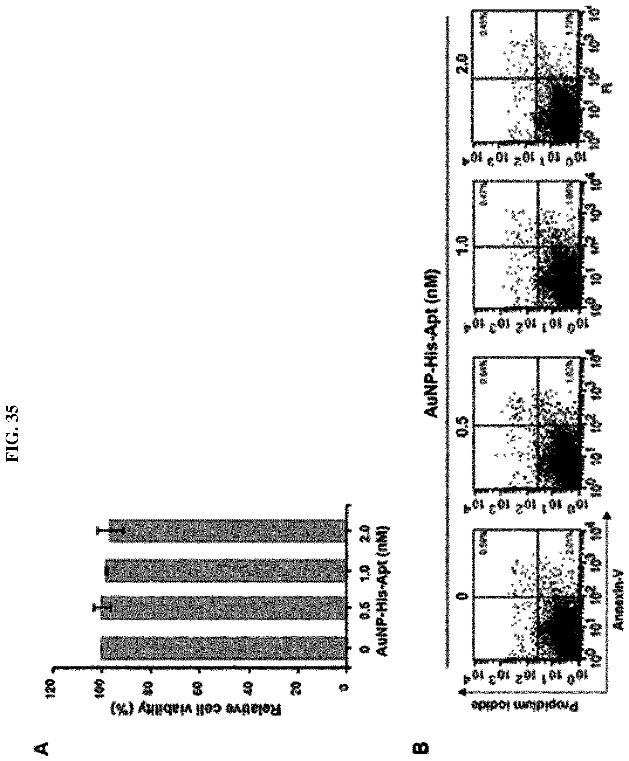

HeLa cells were treated with varying concentrations (0, 0.5, 1.0, and 2 nM) of AuNP-His-Apt and incubated for 12 hours. After the incubation, cell viability was measured and the results are shown in FIG. 35. Results (mean ± SEM) are from three independent experiments performed in triplicate.

Additionally, Annexin V-positive apoptotic cells were confirmed by flow cytometry analysis, and the results are shown in FIG. 35.

As shown in FIG. 35, it was confirmed that AuNP-His-Apt of the present invention had no detectable cytotoxicity.

**Example 11. Confirmation of multiple protein delivery capability of gold nanoparticle-aptamer conjugate**

To confirm multiple protein delivery capability of the gold nanoparticle-aptamer conjugate, the following experiment was performed.

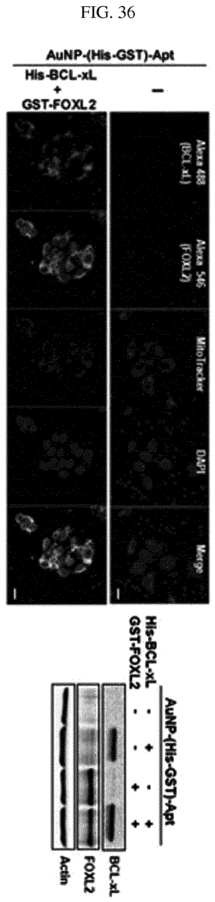

AuNP (AuNP-(His-GST)-Apt) conjugated with both His-Apt and GST-Apt were allowed to react with His-BCL-xL, GST-FOXL2, or with His-BCL-xL and GST-FOXL2 together. HeLa cells were treated with the AuNP conjugates and incubated for 1 hour and immunostained with anti-BCL-xL antibodies or anti-FOXL2 antibodies, and then analyzed using a confocal fluorescence microscope. The results are shown in FIG. 36. Here, DAPI was used for labeling of nuclei, and the cells were imaged using the 40x water immersion objective lens (Scale bar; 20 $\mu$m). Each protein type was identified through detection of Alexa 488-labeled mouse IgG or Alexa 546-labeled-rabbit IgG.

Additionally, western blot analysis was performed using BCL-xL or FOXL2 antibodies, and the results are shown in FIG. 36.

As shown in FIG. 36, it was confirmed that AuNPs conjugated with both His-tag and GST-tag aptamers delivered the two proteins (His-BCL-xL and GST-FOXL2) to appropriate intracellular compartments.

**Example 12. Confirmation of *in vivo* antitumor activity of AuNP-His-Apt-His-BIM**

12-1. Confirmation of changes in tumor volume and weight

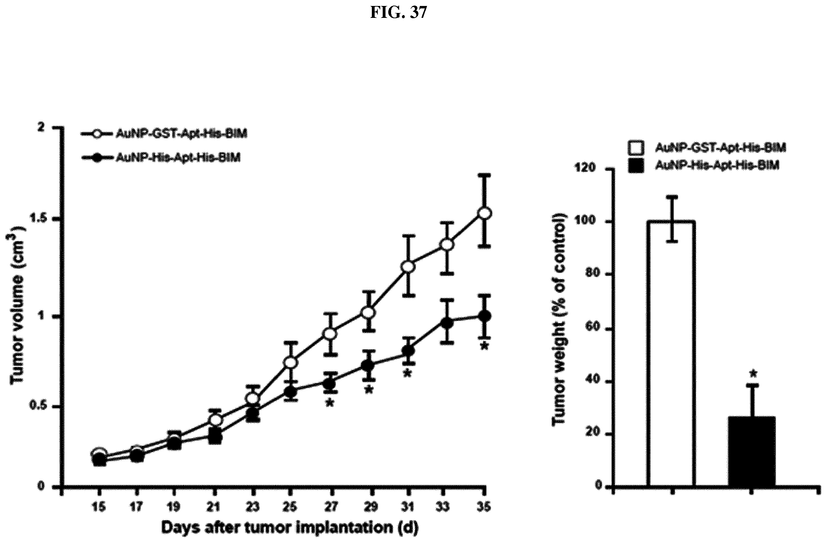

HeLa cells were injected into nude mice to induce cervical cancer and an AuNP-His-Apt-His-BIM complex (0.5 mg/kg) was injected into xenograft tumors every second day and tumor volume over time was calculated by the following equation, and the results are shown in FIG. 37. Meanwhile, the mice of the control group were injected with an AuNP-GST-Apt-His-BIM complex.

[Equation]

$$(\text{length} \times \text{width}^2 \times \pi)/6$$

Also, 30 days after the implantation, the mice were euthanized and tumor weight was measured. The results were shown in FIG. 37. Tumor weight is indicated as the percentage of the control group, data is presented as mean ± SEM and asterisks represent statistical significance (P < 0.05) in comparison with the control group (n=5).

As shown in FIG. 37, both volume and weight of tumor injected with the AuNP-His-Apt-His-BIM showed a significant decrease compared to the control group, therefore from the results, it was found that growth of tumor was suppressed due to the injection of AuNP-His-Apt-His-BIM.

12-2. Confirmation of His-BIM protein delivery to tumor tissues

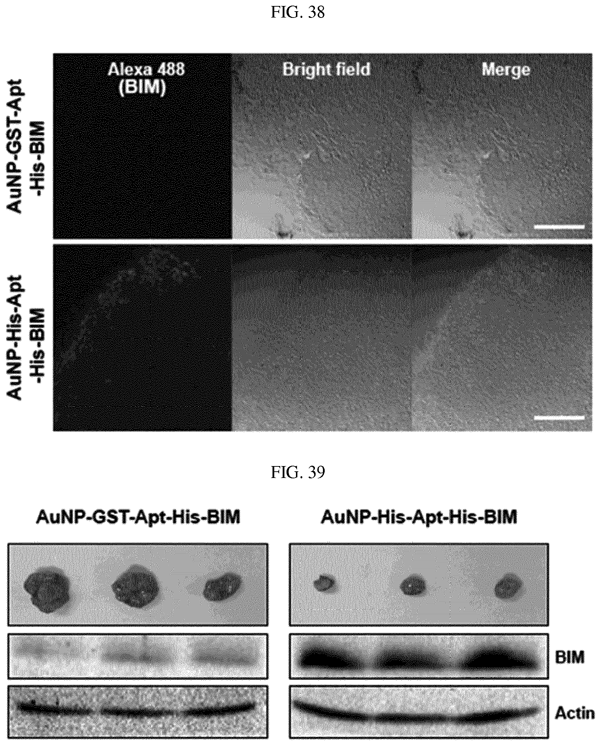

To verify His-BIM protein delivery efficiency to tumor tissues due to an AuNP-His-Apt conjugate, confocal fluorescence microscopy analysis was performed on tumor sections obtained from the mice injected with AuNP-His-Apt loaded with Alexa488-labeled His-BIM protein (Alexa 488-His-BIM). The results are shown in FIG. 38. and the scale bar was 200 $\mu$m.

As shown in FIG. 38, the samples injected with Alexa 488-labeled AuNP-His-Apt-His-BIM show green fluorescence of Alexa 488, and thus it was confirmed that His-BIM protein were delivered to tumor tissues by the AuNP-His-Apt conjugate.

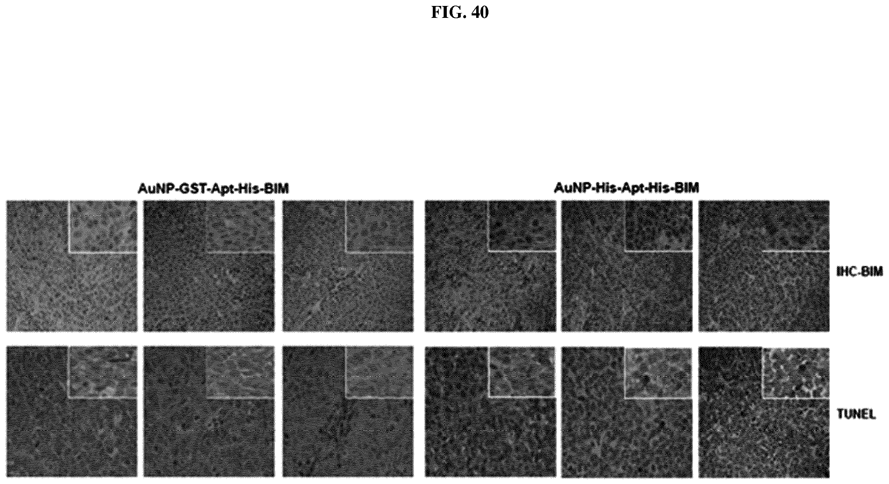

In addition, expression level of BIM protein in tumor tissues was confirmed by western blot and immunohistochemical analyses using anti-BIM antibodies, and the results are shown in FIG. 39 and 40.

As shown in FIG. 39 and FIG. 40, an increase in the BIM protein level was observed in the tumor cells injected with AuNP-His-Apt-His-BIM.

Also, an increase of BIM protein-mediated apoptotic cell death was confirmed by TUNEL analysis and the results are shown in FIG. 40. More specifically, the apoptotic cells were observed using a terminal deoxynucleotidyl transferase-mediated dUTP-biotin nick end labeling (TUNEL) assay kit (Roche, USA).

As shown in FIG. 40, an increase of apoptotic cells was observed in the tumor cells injected with AuNP-His-Apt-His-BIM (lower panel), here, the head of the arrow indicates TUNEL-positive cells.

**Example 13. Confirmation of *in vivo* intracellular protein targeted delivery capability of AuNP-His-Apt**

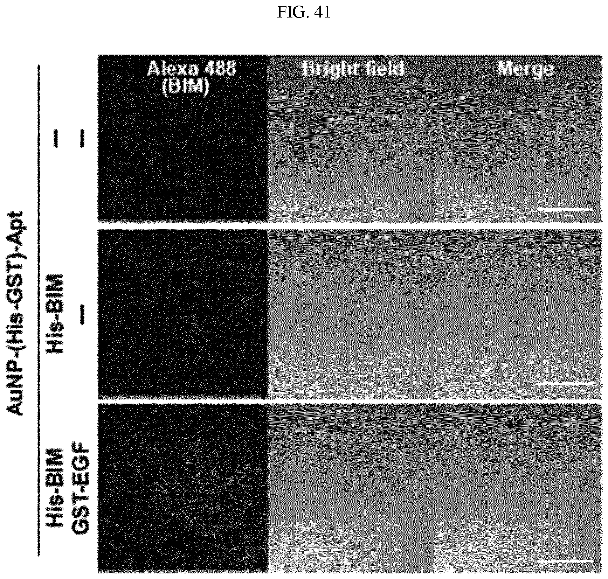

Epidermal growth factor receptor (EGFR)-overexpressing A431 human epidermoid carcinoma cells were injected intraperitoneally into nude mice to allow to form xenograft tumors. Ten days after injection of the cells, AuNP-(His-GST)-Apt loaded with His-BIM only, or AuNP-(His-GST)-Apt (0.5mg/kg of body weight) loaded with both His-BIM and GST-EGF were injected via tail vein injection into the tumor-bearing mice. Here, His-BIM and GST-EGF were labeled with Alexa 488 and Texas Red, respectively. 12 hours after the injection, the mice were euthanized and organs (brain, liver, spleen and ovaries) were retrieved, and sections of the xenograft tumors were observed using a confocal fluorescence microscope. The results are shown in FIG. 41 and the scale bar was 200 μm. As shown in FIG. 41, a stronger green signal reflecting efficient BIM protein delivery was observed upon delivery of AuNP-Apt conjugated with Alexa 488-loaded BIM along with Texas Red-loaded EGF whereas less BIM delivery was detected in the absence of EGF protein

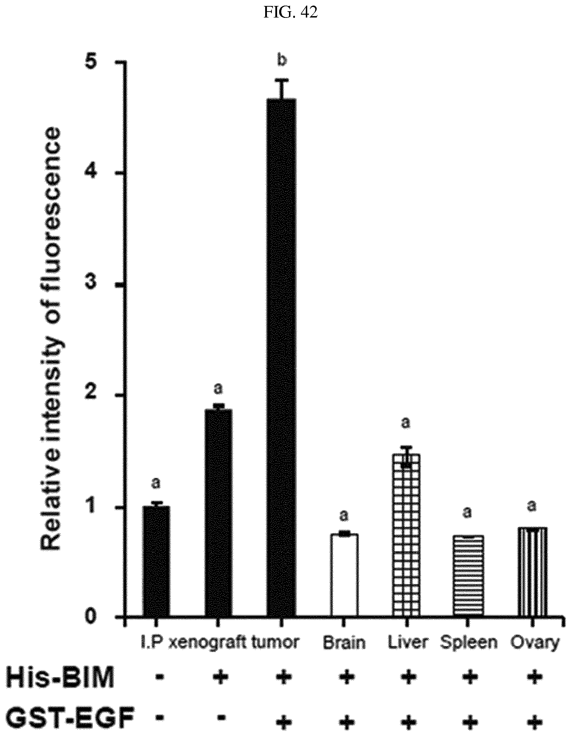

Also, relative intensities of fluorescence in sections of tumors and organs were measured using Image J software, and the results are shown in FIG. 42. Results (mean ± SEM) were obtained from three mice. Statistically significant values are indicated as P < 0.05.

As shown in FIG. 42, significant increased BIM absorption was observed in EGFR-expressing tumors, compared to tumor tissues and other organs such as the brain, liver, spleen and ovaries.

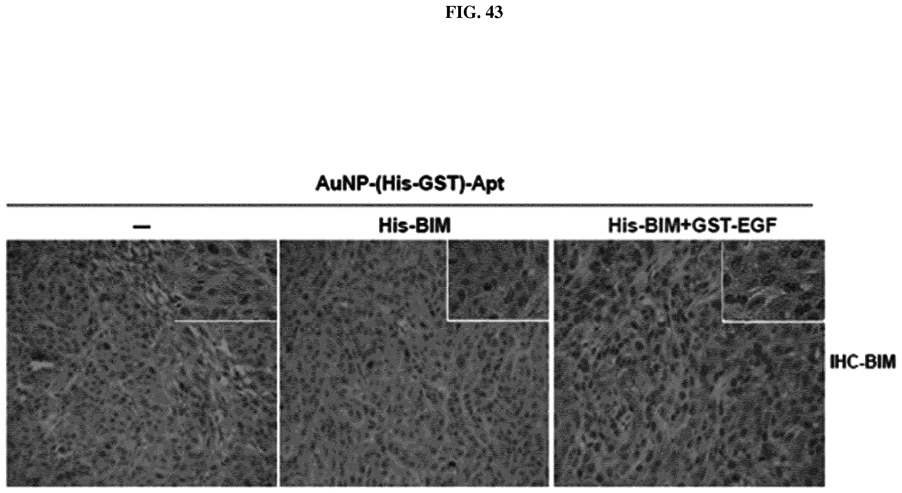

In addition, immunohistochemical analysis was performed on tumor tissues by using anti-BIM antibodies and the results are shown in FIG. 43.

From these results, it was found that the gold nanoparticle-aptamer conjugate of the present application was capable of *in vivo* targeted delivery of proteins.

**Example 14. Confirmation of biodistribution of AuNP-His-Apt-Alexa488-His-BIM complex**

14-1. Systemic distribution of His-BIM protein by AuNP-His-Apt

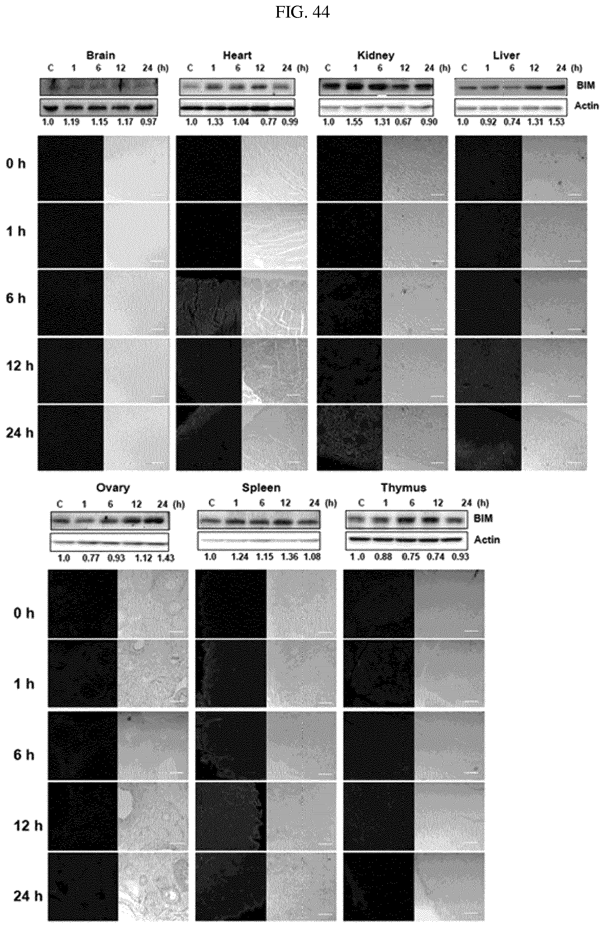

AuNP-His-Apt-Alexa488-His-BIM prepared by reacting 10 μg of Alexa488-His-BIM with AuNP-His-Apt, was intravenously injected into rats (1 mg/kg), and organ samples (brain, heart, kidney, liver, ovaries, spleen and thymus) were collected at each time point (0, 1, 6, 12, 24 hours). The samples were analyzed using a confocal microscope and the results are shown in FIG. 44. Representative fluorescence images indicate the presence of Alexa488-His-BIM in tissues of the brain, heart, kidney, liver, ovaries, spleen and thymus at each time point and the scale bar was 200 μm.

Also, western blot analysis was performed on the organ lysates using anti-BIM and anti-β-actin antibodies, and the results are shown in FIG. 44.

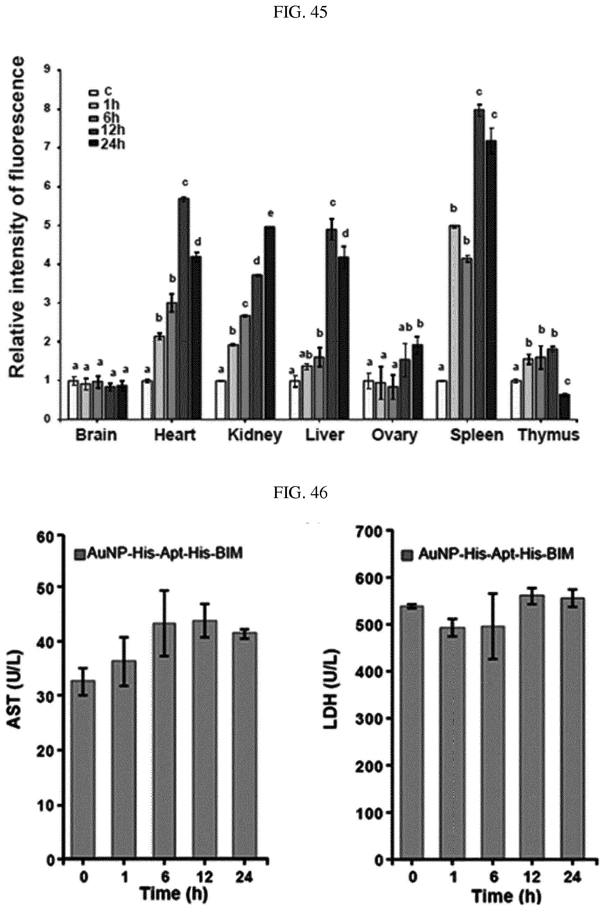

In addition, relative intensities of fluorescence in tissues were measured using Image J software, and the results are shown in FIG. 45. The values are shown by setting intensities of fluorescence from organ treated with AuNP-His-apt as 1 (n = 6/7, respectively). Statistical significance values are indicated as P < 0.05.

14-2. Confirmation of changes in AST and LDH release level by AuNP-His-Apt-Alexa488-His-BIM

AuNP-His-Apt-His-BIM prepared by reacting 10 μg of His-BIM and AuNP-His-Apt, was intravenously injected (1 mg/kg) into 4 week-old female rats and 24 hours after the injection, the levels of aspartate transaminase (AST) and lactate dehydrogenase (LDH) were measured by a colorimetric assay. More specifically, serum levels of AST and LDH were measured using the IDTox™ Aspartate Transaminase (AST) Color Endpoint Assay Kit (ID Labs™ Inc, Canada) and IDTox™ Lactate Dehydrogenase (LDH) Color Endpoint Assay Kit (ID Labs™ Inc) respectively, and the results are shown in FIG. 46. The results are indicated as mean ± SEM (n=3 rat, P value not significant).

[Industrial Applicability]

A protein delivery carrier according to the present invention can effectively deliver a protein into cells by specifically binding a variety of tagged aptamers or protein-specific aptamers conjugated with a gold nanoparticle to a protein to be delivered. Also, the protein delivery carrier is harmless to the human body by using gold nanoparticles with a very low cytotoxicity and expected to be useful for diagnosis or therapy of a disease by reflecting light in various wavelengths to easily detect a location in the cells.

<110> Chung-Ang University Industry-Academy Cooperation Foundation

<120> GOLD NANOPARTICLE-APTAMER CONJUGATES-BASED PROTEIN DELIVERY SYSTEM AND PREPARATION METHOD THEREOF

<130> PCT01887

<160> 2

<170> KopatentIn 2.0

<210> 1
<211> 50
<212> DNA
<213> Artificial Sequence

<220>
<223> his-tag DNA aptamer

<400> 1
gctatgggtg gtctggttgg gattggcccc gggagctggc
aaaaaaaaaa        50

<210> 2
<211> 50
<212> DNA
<213> Artificial Sequence

<220>
<223> GST-tag DNA aptamer

<400> 2
ctgccccgct atagaacacc cgttgggcaa atgtgttcga

aaaaaaaaaa    50

use in therapy.

### Claims

**1.** A protein delivery carrier, comprising:

a gold nanoparticle; and
an aptamer conjugated to a surface of the gold nanoparticle
wherein the aptamer is selected from an aptamer that has SEQ ID NO: 1 and can specifically bind to a histidine-tag and/or an aptamer that has SEQ ID NO: 2 and can specifically bind to a glutathione S-transferase (GST)-tag.

**2.** The protein delivery carrier of claim 1, wherein the gold nanoparticle has a size of 10 to 20 nm.

**3.** The protein delivery carrier of claim 1 or claim 2, wherein the aptamer can specifically bind to a histidine tag and has SEQ ID NO:1.

**4.** The protein delivery carrier of claim 1 or claim 2, wherein the aptamer can specifically bind to a glutathione S-transferase (GST) tag and has SEQ ID NO:2.

**5.** The protein delivery carrier of any one of claims 1-4, wherein both an aptamer which can specifically bind to a histidine-tag and has SEQ ID NO: 1 and an aptamer which can specifically bind to a glutathione S-transferase (GST)-tag and has SEQ ID NO: 2 are conjugated to a surface of the gold nanoparticle.

**6.** A method of preparing the protein delivery carrier of any one of claims 1-5, comprising:
conjugating a gold nanoparticle with one or more aptamers, wherein the one or more aptamers are selected from an aptamer that has SEQ ID NO: 1 and can specifically bind to a histidine-tag and/or an aptamer that has SEQ ID NO: 2 and can specifically bind to a glutathione S-transferase (GST)-tag.

**7.** An isolated protein delivery complex, comprising;
a gold nanoparticle;
an aptamer conjugated to a surface of the gold nanoparticle; and
a protein conjugated to the aptamer,
wherein the aptamer is selected from an aptamer that has SEQ ID NO: 1 and can specifically bind to a histidine-tag and/or an aptamer that has SEQ ID NO: 2 and can specifically bind to a glutathione S-transferase (GST)-tag, and
wherein the protein is labeled with a histidine-tag and/or a glutathione S-transferase (GST)-tag.

**8.** A protein delivery complex according to claim 7 for

### Patentansprüche

**1.** Proteinlieferträger, umfassend:

ein Goldnanopartikel; und
ein Aptamer, das an eine Oberfläche des Goldnanopartikels konjugiert ist
wobei das Aptamer aus einem Aptamer ausgewählt ist, das SEQ ID NR: 1 aufweist und spezifisch an ein Histidin-Tag und/oder ein Aptamer binden kann, das SEQ ID NR: 2 aufweist und spezifisch an ein Glutathion-S-Transferase (GST)-Tag binden kann.

**2.** Proteinlieferträger nach Anspruch 1, wobei das Goldnanopartikel eine Größe von 10 bis 20 nm aufweist.

**3.** Proteinlieferträger nach Anspruch 1 oder Anspruch 2, wobei das Aptamer spezifisch an ein Histidin-Tag binden kann und SEQ ID NR: 1 aufweist.

**4.** Proteinlieferträger nach Anspruch 1 oder Anspruch 2, wobei das Aptamer spezifisch an ein Glutathion-S-Transferase (GST)-Tag binden kann und SEQ ID NR: 2 aufweist.

**5.** Proteinlieferträger nach einem der Ansprüche 1-4, wobei sowohl ein Aptamer, das spezifisch an ein Histidin-Tag binden kann und SEQ ID NR: 1 aufweist, als auch ein Aptamer, das spezifisch an ein Glutathion-S-Transferase (GST)-Tag binden kann und SEQ ID NR: 2 aufweist, an eine Oberfläche des Goldnanopartikels konjugiert sind.

**6.** Verfahren zum Zubereiten des Proteinlieferträgers nach einem der Ansprüche 1-5, umfassend:
Konjugieren eines Goldnanopartikels mit einem oder mehr Aptameren, wobei das eine oder die mehr Aptamere aus einem Aptamer, das SEQ ID NR: 1 aufweist und spezifisch an ein Histidin-Tag binden kann, und/oder einem Aptamer, das SEQ ID NR: 2 aufweist und spezifisch an ein Glutathion-S-Transferase (GST)-Tag binden kann, ausgewählt sind.

**7.** Isolierter Proteinlieferkomplex, umfassend:

ein Goldnanopartikel;
ein Aptamer, das an eine Oberfläche des Goldnanopartikels konjugiert ist; und
ein Protein, das an das Aptamer konjugiert ist, wobei das Aptamer aus einem Aptamer, das SEQ ID NR: 1 aufweist und spezifisch an ein Histidin-Tag binden kann, und/oder einem Aptamer, das SEQ ID NR: 2 aufweist und spezifisch an ein Glutathion-S-Transferase (GST)-Tag binden kann, ausgewählt ist, und wobei das Protein mit einem Histidin-Tag und/oder einem Glutathion-S-Transferase (GST)-Tag markiert ist.

8. Proteinlieferkomplex nach Anspruch 7 zur Verwendung in Therapie.

**Revendications**

1. Vecteur d'administration de protéine, comprenant :

    une nanoparticule d'or ; et
    un aptamère conjugué à une surface de la nanoparticule d'or
    dans lequel l'aptamère est choisi parmi un aptamère qui a SEQ ID NO : 1 et peut spécifiquement se lier à une étiquette d'histidine et/ou un aptamère qui a SEQ ID NO : 2 et peut se lier spécifiquement à une étiquette de glutathion-S-transférase (GST).

2. Vecteur d'administration de protéine selon la revendication 1, dans lequel la nanoparticule d'or a une taille de 10 à 20 nm.

3. Vecteur d'administration de protéine selon la revendication 1 ou la revendication 2, dans lequel l'aptamère peut se lier spécifiquement à une étiquette d'histidine et a SEQ ID NO : 1.

4. Vecteur d'administration de protéine selon la revendication 1 ou la revendication 2, dans lequel l'aptamère peut se lier spécifiquement à une étiquette de glutathion-S-transférase (GST) et a SEQ ID NO : 2.

5. Vecteur d'administration de protéine de l'une quelconque des revendications 1 à 4, dans lequel un aptamère qui peut se lier spécifiquement à une étiquette d'histidine et a SEQ ID NO : 1 et un aptamère qui peut se lier spécifiquement à une étiquette de glutathion-S-transférase (GST) et a SEQ ID NO : 2 sont conjugués à une surface de la nanoparticule d'or.

6. Procédé de préparation du vecteur d'administration de protéine selon l'une quelconque des revendications 1 à 5, comprenant les étapes consistant à : conjuguer une nanoparticule d'or avec un ou plusieurs aptamères, dans lequel les un ou plusieurs aptamère sont choisis parmi un aptamère qui a SEQ ID NO : 1 et peut se lier spécifiquement à une étiquette d'histidine et/ou un aptamère qui a SEQ ID NO : 2 et peut se lier spécifiquement à une étiquette de glutathion-S-transférase (GST).

7. Complexe d'administration de protéine isolé, comprenant ;
    une nanoparticule d'or ;
    un aptamère conjugué à une surface de la nanoparticule d'or ; et
    une protéine conjuguée à l'aptamère,
    dans lequel l'aptamère est choisi parmi un aptamère qui a SEQ ID NO : 1 et peut se lier spécifiquement à une étiquette d'histidine et/ou un aptamère qui a SEQ ID NO : 2 et peut se lier spécifiquement à une étiquette de glutathion-S-transférase (GST), et
    dans lequel la protéine est marquée avec une étiquette d'histidine et/ou une étiquette de glutathion-S-transférase (GST).

8. Complexe d'administration de protéine selon la revendication 7 pour une utilisation en thérapie.

FIG. 1

FIG. 2

Negative(HeLa cell)

AuNP-His aptamer

His-AcrA (1 µg/µl)

AcrA (1 µg/µl)

AuNP-His aptamer +His–AcrA (0.5 µg/µl)

AuNP-His aptamer +AcrA (0.5 µg/µl)

AuNP-His aptamer +His-AcrA (1 µg/µl)

AuNP-His aptamer +AcrA (1 µg/µl)

FIG. 3

Negative(HeLa cell)

AuNP-His aptamer

His-AcrA

AcrA

AuNP-His aptamer +His-AcrA (0.5μg/μl)

AuNP-His aptamer +AcrA (0.5 μg/μl)

AuNP-His aptamer +His-AcrA (1 μg/μl)

AuNP-His aptamer +AcrA (1 μg/μl)

FIG. 4

FIG. 5

Anti-His

Anti-AcrA

1. AuNP-Aptamer
2. AuNP-Aptamer +His-AcrA (0.5 µg/µl)
3. AuNP-Aptamer +His-AcrA (1 µg/µl)
4. AuNP-Aptamer +AcrA (0.5 µg/µl)
5. AuNP-Aptamer +AcrA (1 µg/µl)

FIG. 6

FIG. 7

Negative(HeLa cell)

AuNP-His aptamer

His-BCL-XL (1 µg/µl)

BCL-XL (1 µg/µl)

AuNP-His aptamer +His–BCL-XL (0.5 µg/µl)

AuNP-His aptamer +BCL-XL (0.5 µg/µl)

AuNP-His aptamer +Hiis-BCL-XL (1 µg/µl)

AuNP-His aptamer +BCL-XL (1 µg/µl)

FIG. 8

FIG. 9

Anti-Bcl-xL

Anti-GAPDH

1. AuNP-Apt
2. AuNP-Apt-Bcl-xL (5 µg/µl)
3. AuNP-Apt-His-Bcl-xL (5 µg/µl)

FIG. 10

24

EP 2 995 300 B1

FIG. 11

AuNP-His-apt

Bcl-xL

His-Bcl-xL

+

−

FIG. 12

1    2    3    4

A

B

C

25

FIG. 13

FIG. 14

## AuNP-GST aptamer

## AuNP-GST aptamer – GST-FoxL2 (5 µg/µl)

## AuNP-GST aptamer – FoxL2 (5 µg/µl)

FIG. 15

**Anti-FoxL2**

**Anti-GAPDH**

1. **AuNP-GST-Apt**
2. **AuNP-GST-Apt-FoxL2 (5 µg/µl)**
3. **AuNP-GST-Apt-GST-FoxL2 (5 µg/µl)**

FIG. 16

**AuNP-GST-Apt**

**GST-FOXL2** —

FIG. 17

FIG. 18

FIG. 19

FIG. 20

FIG. 21

FIG. 22

FIG. 23

FIG. 24

FIG. 25

FIG. 26

FIG. 27

FIG. 28

FIG. 29

FIG. 30

FIG. 31

FIG. 32

FIG. 33

FIG. 34

FIG. 35

EP 2 995 300 B1

FIG. 36

**FIG. 37**

FIG. 38

FIG. 39

FIG. 40

FIG. 41

FIG. 42

FIG. 43

AuNP-(His-GST)-Apt

—      His-BIM      His-BIM+GST-EGF

IHC-BIM

FIG. 44

FIG. 45

FIG. 46

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2007134665 A

**Non-patent literature cited in the description**

- **YU JIN KIM ; SANG-MI RYOU ; SUDEOK KIM ; JI-HYUN YEOM ; MIN SU HAN ; KANGSEOK LEE ; MAENG-JE SEONG.** Enhanced protein-mediated binding between oligonucleotide gold nanoparticle composites and cell surfaces: co-transport of proteins and composites. *J. Mater. Chem.,* 2012, vol. 22, 25036
- **OZNUR KÖKPINAR et al.** *Biotechnology and Bioengineering,* 2011, vol. 108 (10), 2371-2379
- **DANIELS et al.** *Analytical Biochemistry,* 2002, vol. 305 (2), 214-226
- **GHOSH et al.** *Advanced Drug Delivery Reviews,* 2008, vol. 60 (11), 1307-1315
- **JOSHI et al.** Langmuir. American Chemical Society, 2006, vol. 22, 300-305
- **YEOM, J.H. et al.** Inhibition of Xenograft Tumor Growth by Gold Nanoparticle-DNA Oligonucleotide Conjugates-Assisted Delivery of BAX mRNA. *PLoS One,* 2013, vol. 8, e75369
- **KIM, J.H. et al.** Differential apoptotic activities of wild-type FOXL2 and the adult-type granulosa cell tumor-associated mutant FOXL2 (C134W). *Oncogene,* 2010, vol. 30, 1653-1663